# EUROPEAN PATENT APPLICATION

(11) **EP 3 407 067 A1**
(43) Date of publication of application: **28.11.2018**
(21) Application number: 17172184.8
(22) Date of filing: 22.05.2017
(51) Int. Cl.: G01N 33/68

(54) **METHOD OF DETECTING COLITIS ULCEROSA BY DETECTION OF AUTOANTIBODIES**

(71) Applicant: AIT Austrian Institute of Technology GmbH, 1220 Wien (AT)
(72) Inventor: WEINHÄUSEL, Andreas, 7311 Neckenmarkt (AT); GROPP, Roswitha, 80336 Munich (DE); BEIKIRCHER, Gabriel, 1190 Vienna (AT); SCHÖNTHALER, Silvia, 2563 Pottenstein (AT); SOLDO, Regina, 1050 Vienna (AT); NOEHAMMER, Christa, 1100 Vienna (AT); VIERLINGER, Klemens, 1090 Vienna (AT); KULOVICS, Ronald, 3413 Hintersdorf (AT); SIEBECK, Matthias, 82131 Stockdorf (DE)
(74) Representative: Sonn & Partner Patentanwälte

(57) **Abstract**

The present invention provides a method of predicting emergence of colitis ulcerosa or detecting a predisposition to colitis ulcerosa in a subject comprising providing a sample from the subject, and detecting autoantibodies of the subject directed against antigens of colitis ulcerosa in the sample.

## Description

### Background of the invention

Colitis ulcerosa (also termed "ulcerative colitis", UC) is, besides Crohn's disease (CD), one of the main types of inflammatory bowel disease (IBD), characterized by chronic inflammation of the gastrointestinal tract due to an immune response targeting the gut microbiota (Prideaux et al., Inflamm Bowel Dis. 2012;18(7):1340-55). On the serological level, certain antibody-based biomarkers for diagnosis of IBD and for further differential diagnosis have already been investigated. Targets of currently tested antibodies are epitopes or bacterial and fungal antigens (Tontini et al., World J Gastroenterol. 2015; 21(1): 21-46).

US 5,968,741 relates to the detection of antibodies directed against *Saccharomyces cerevisiae* in the diagnosis of UC.

US 6,074,835 relates to binding histone H1 to perinuclear anti-neutrophil cytoplasmic antibody (pANCA) in identifying pANCA-positive subtypes of UC in patients that have already been identified with US. pANCA itself is not suitable to diagnose UC in general, irrespective of this subtype, since it is only expressed in 10%-15% of UC cases while it is expressed in 60%-70% of CD and could therefore lead to a misdiagnosis of CD instead of UC (Mitsuyama et al., World J Gastroenterol 2016; 22(3): 1304-1310). A detection of pANCA in a subject that has not already been diagnosed with UC, UC cannot be diagnosed based on pANCA.

Clinically most important utility of serological biomarkers is to evaluate the aggressive risks of disease phenotype, complications or surgery requirement, predict prognosis of the disease and distinguish CD from UC. However, no markers with qualities that are satisfactory and provide good clinical utility and high performance in respect to diagnosis and treatment of UC/IBD have been identified. There remains a need for reliable diagnosis of UC, especially irrespective of subtypes of already diagnosed UC. The prior art also completely fails to address the problem of pre-diagnosis, i.e. to evaluate the risk of developing UC in healthy or non-UC subjects.

### Summary of the invention

The invention provides a method of predicting emergence of colitis ulcerosa, detecting a predisposition to colitis ulcerosa (both also referred to herein as risk of developing colitis ulcerosa) or of diagnosing colitis ulcerosa in a subject comprising providing a sample from the subject, and detecting autoantibodies specific of colitis ulcerosa of the subject in the sample. In other words, the invention provides a method of predicting emergence of colitis ulcerosa, detecting a predisposition to colitis ulcerosa or of diagnosing colitis ulcerosa in a subject comprising providing a sample from the subject, and detecting autoantibodies of the subject directed against antigens of colitis ulcerosa in the sample.

The present invention provides a method of presymptomatic detection of colitis ulcerosa or monitoring intestinal bowel disease, preferably colitis ulcerosa in a subject comprising providing an immunoglobulin-containing sample from the subject, and detecting changed reactivities of (auto)-antibodies of the subject directed against characteristic antigens.

The invention also relates to a method of treating a subject having colitis ulcerosa or being at risk of developing colitis ulcerosa, comprising detecting colitis ulcerosa or the risk thereof according to the invention and performing a colitis ulcerosa treatment such as administering a colitis ulcerosa medication, preferably an anti-inflammatory medicament.

In a further aspect, the invention relates to a kit of diagnostic agents suitable to detect autoantibodies against any antigen or antigen combination as described herein, wherein said diagnostic agents comprise antigens suitable to bind autoantibodies in a sample.

All aspects relate to each other and any embodiment described for one aspect commensurately also relates to all other aspects. E.g. the antigens described for the method or the means for the method can be included in the kit. The kit or any part thereof can be used in the inventive methods.

### Detailed description of the invention

The present invention provides a new diagnostic class of analyses in the detection, in particular in case of predisposition and prognosis evaluation, of colitis ulcerosa markers. The new analytes are autoantibodies of a subject that can be found in a sample from the subject, like blood, serum, saliva or urine. Autoantibodies are self-antibodies that target antigens of the subject itself, i.e. not the subject's bacterial or fungal microbiota but the subject's own cells or cell products, like proteins, e.g. cell surface proteins. Surprisingly, a subject suffering from colitis ulcerosa also develops an immune reaction against such self-antigens. Even more surprising, such antibodies against self-antigens are distinctive enough to allow diagnostic and prognostic (predisposition) analyses.

Accordingly, the invention provides a method of predicting emergence of colitis ulcerosa, detecting a predisposition to colitis ulcerosa or of diagnosing colitis ulcerosa in a subject comprising providing a sample from the subject, and detecting autoantibodies of the subject in the sample, with the autoantibodies being specific of colitis ulcerosa and/or are directed against antigens of colitis ulcerosa in the sample.

Since literature is non-uniform in the use of the term "autoantibody", it is again emphasized that according to the invention an autoantibody is an antibody against an antigen expressed by the subject organism, e.g. if the subject is human, then the antigen is a human antigen. Antibodies against microflora, such as ASCA (anti-Saccharomyces cerevisiae antibody), ACCA (anti-chitobioside carbohydrate antibody) and ALCA (antilaminaribio-side carbohydrate antibodies) are not considered autoantibodies according to the invention. They are anti-microflora-antibodies. Detection of anti-microflora-antibodies may be excluded according to the invention or they can be detected in addition to the inventive autoantibodies. Preferred anti-microflora-antibodies that can be detected according to the invention in the diagnosis of UC or in estimating the risk thereof are anti-E.coli antibodies.

Herein, it is shown that an autoantibody response is an early event in UC, which allows presymptomic and early diagnosis, and prognosis. UC-auto-antigenicity is affecting a plethora of self-antigens with specific human protein antigens highly correlated with disease activity. Diagnosis by autoantibodies could minimize the use of endoscopic and radiologic examinations and allows clinicians to implement treatment plans designed to improve the long-term prognosis of patients with UC.

Herein "patient" and "subject" are essentially interchangeably used. Patient may mean that the subject has a disease or is or should be in treatment.

"Markers", "disease markers" and "antigens" are referred to herein as the target of the inventive autoantibodies. Although the autoantibodies are detected in the inventive methods, it is clearer to refer to the specificity of the antibodies, i.e. the inventive markers instead of the autoantibodies. Such references here shall be understood as targets of the autoantibodies that are detected by the inventive methods. An antigen is any substance that causes an immune system to produce antibodies against it. When referring to an antigen or marker in the context of autoantibody target, autoantibodies are described that bind these full-length or complete proteins. Such autoantibodies are usually reactive against antigenic fragments of the complete proteins, i.e. proteins or peptides that contain an epitope and hence bind to the antibody. Such binding targets of the autoantibody are also antigens.

Especially preferred is an early risk estimation of UC. Risk estimation includes predicting emergence of colitis ulcerosa, detecting a predisposition to colitis ulcerosa or determining the risk of developing colitis ulcerosa. All of these aspects of risk estimation can be determined by any inventive method for any risk estimation with little calculative adaptation of the output data format, if needed.

In particular embodiments of the invention "early risk" estimation demands that the subject has not been diagnosed with colitis ulcerosa or the subject is not of suffering from colitis ulcerosa at the time of the inventive diagnosis or the procurement of the sample for the inventive diagnosis from the subject. The inventive method may be a prediction of emergence of colitis ulcerosa or a detection of a predisposition to colitis ulcerosa.

"Diagnosis" for the purposes of this invention means the positive determination of colitis ulcerosa by means of the marker proteins according to the invention as well as the assignment of the patients to colitis ulcerosa. The term "diagnosis" covers medical diagnostics and examinations in this regard, in particular in-vitro diagnostics and laboratory diagnostics, likewise serological analysis, proteomics and peptide blotting. Further tests can be necessary to be sure and to exclude other diseases. The term "diagnosis" therefore likewise covers the differential diagnosis of colitis ulcerosa by means of the marker proteins according to the invention and the risk or prognosis of colitis ulcerosa.

The invention and any marker described herein can be used to distinguish between normal conditions and colitis ulcerosa or risk of colitis ulcerosa. A positive result in distinguishing said indications can prompt a further risk or disease test, in particular more invasive tests than a blood or serum test such as a biopsy.

The autoantibodies being specific of colitis ulcerosa and/or being directed against antigens of colitis ulcerosa in the sample essentially mean the same thing, i.e. the autoantibodies or their amounts are characteristic of colitis ulcerosa. The antigens of the autoantibodies are usually normal self-antigens that can also be found in healthy subjects with no risk of colitis ulcerosa. In colitis ulcerosa, or in a predisposition thereto, these antigens are targeted by autoantibodies in increased amounts. This targeting makes the antigens or markers "specific" of colitis ulcerosa. Also, the autoantibodies can be found in healthy subjects, but as said the autoantibody amount is significantly increased in colitis ulcerosa. This increase allows the inventive diagnosis or risk estimation. In preferred embodiments, it is also not a single autoantibody against a single antigen (of colitis ulcerosa) that is detected, instead preferably a total antibody response against an antigen of colitis ulcerosa that is directed. Such an antibody response is defined by the antibodies in the sample. These antibodies may be only of a given antibody class, e.g. IgG, or require at least such an antibody class as described in more detail herein.

To detect an (auto)antibody in a sample it is possible to use marker proteins as binding agents and subsequently to detect bound antibodies bound to the marker proteins. It is not necessary to use the entire marker proteins but it is sufficient to use antigenic fragments that are bound by the antibodies. "Antigenic fragment" herein relates to a fragment of the marker protein that causes an immune reaction against said marker protein in a human. Such antigenic fragments may be antigenic in a plurality of humans, such as at least 5, or at least 10 individuals. The markers may be immobilized to allow easy handling and binding assays to determine the autoantibodies. Accordingly, the autoantibody determination may comprise binding to the antigens and detecting binding events. Preferably, herein the antigens are immobilized on a solid surface. A solid surface may be any surface commonly used in antigen-antibody binding assays, such as glass or synthetic material surface. Examples are microarray surfaces or microtiter wells. Other methods that can be used according to the invention do not require a solid surface such as immune-PCR.

Autoantibodies that are bound to an antigen of colitis ulcerosa can be detected or visualized by any known means. For example detection of autoantibodies can be achieved during or after binding to the respective antigen. For detection or visualization, the use of a label is common, such as by an optical label such as a fluorescence label or a chromophoric label. Other labels include radio labels, enzymatic labels or a nucleic acid sequence tag. Enzymatic labels may provide an enzyme or a substrate for a detectable reaction. In one embodiment, antigen-bound antibodies are bound by antibody specific reagents, such as a secondary antibody that may be labelled. In case the auto-antibody is IgG, a second antibody may be specific for the constant part (Fc) of an IgG of the subject.

Usually the result of the autoantibody detection from a sample of a subject is compared to a reference detection value. Detection values are usually quantification signals that indicate a concentration or amount in a given comparative sample. E.g. the autoantibody detection may be a determination of autoantibody amounts in comparison to antibody amounts in healthy subjects that did not develop colitis ulcerosa. In preferred embodiments, the healthy subject did not develop colitis ulcerosa within at least a year - this term of a UC disease-free period is usually sufficient to indicate significant diagnostic differences in autoantibody levels or amounts to be used according to the invention. Such healthy subjects can be used a good healthy controls. If established UC shall be detected, it is also possible to use any healthy control - even those that have developed US later as this still shows significant marker differences. The use of the sample or control may determine the selection of indicative markers, but is easily possible within the present invention, which shows simple and reproducible comparison methods.

The control determination can be done at any time. It is for example possible to perform the control in the same set-up as the determination of the sample from the subject to be under investigation. Alternatively, it is also possible to use pre- or post-obtained control data. Such data is comparative due to the similar or comparable experimental set-up being used.

The control samples can be used to obtain a marker dependent signal pattern as indication classifier. Such a signal pattern can be obtained by routine statistical methods, such as binary tree methods. Common statistical methods calculate a (optionally multi-dimensional) vector within the multitude of control data signal values as diagnostically significant distinguishing parameter that can be used to distinguish one or more indications from other one or more indications. The step usually comprises the step of "training" a computer software with said control data. Such pre-obtained training data or signal data can be provided on a computer-readable medium to a practitioner who performs the inventive diagnosis. Preferably, the method comprises optimizing the selection process, e.g. by selecting alternative or additional markers and repeating said comparison with the controls signals, until a specificity and/or sensitivity of at least 75% is obtained, preferably of at least 80%, at least 85%, at least 90%, at least 95%.

Preferably the autoantibodies are detected in a blood or serum sample from the patient or subject. Preferably the entire antibody content of the sample is normalized (e.g. diluted to a pre-set concentration) and detected, e.g. contacted to antigens for detection and binding assays. Preferably the IgG, IgM, IgD, IgA or IgE antibody fraction, is exclusively used. Preferred antibodies that are detected are IgG. Preferably the subject is a mammal, in particular a human.

Although the detection of a single marker can be sufficient to indicate a risk for UC or diagnose UC, it is preferred to use more than one marker, e.g. 2, 3, 4, 5, 6, 7, 8, 9, 10, 11 or 12 or more markers in combination, especially if combined with statistical analysis. Means for statistical analysis can e.g. be provided on a computer-readable memory device for operation on a computer. Such analysis means, e.g. a computer program, may be capable to analyse marker measurement data and comparison to evaluate a risk of or diagnose UC. From a diagnostic point of view, a single autoantigen based diagnosis can be improved by increasing sensitivity and specificity by using a panel of markers where multiple auto-antibodies are being detected simultaneously. Auto-antibodies in a sample can be detected by binding to the marker proteins or their antigenic fragments or epitopes. Accordingly, it is preferred that the antigens of colitis ulcerosa are selected by testing antibody-binding of patient derived immunoglobulins against antigens by multiplexed analysis.

As said, binding events can be detected as known in the art, e.g. by using labelled secondary antibodies. Such labels can be enzymatic, chromophoric, fluorescent, radioactive or a nucleic acid sequence tag. Such labels can also be provided on the binding means, e.g. the antigens as described in the previous paragraph. Nucleic acid sequence tags are especially preferred labels since they can be used as sequence code that not only leads to quantitative information but also to a qualitative identification of the detection means (e.g. antibody with certain specificity). Nucleic acid sequence tags can be used in known methods such as Immuno-PCR. In multiplex assays, usually qualitative information is tied to a specific location, e.g. spot on a microarray. With qualitative information provided in the label, it is not necessary to use such localized immunoassays. In is possible to perform the binding reaction of the analyte and the detection means, e.g. the serum antibody and the labelled antigen, independent of any solid supports in solution and obtain the sequence information of the detection means bound to its analyte. A binding reaction allows amplification of the nucleic acid label in a detection reaction, followed by determination of the nucleic acid sequence determination. With said determined sequence the type of detection means can be determined and hence the marker (analyte, e.g. serum antibody with UC associated antigen specificity).

The antigens of colitis ulcerosa can be selected from antigens that are bound by autoantibodies of individuals that did develop colitis ulcerosa, wherein the autoantibodies bound to the antigens are collected at a time where the individuals did not yet suffer from colitis ulcerosa. Such markers indicate prognosis cases. When identifying markers, such markers may be compared with healthy samples (of subjects that also did not develop UC). Comparing samples of subjects that were both healthy at the time of autoantibody collection but with one subject (or group of subjects) developing UC and another subject (or group of subjects) that did not develop UC allows the selection of markers that are of diagnostic value in risk estimation of developing UC. Developing or not developing UC may be in a preselected time frame, e.g. within 4 months, within 6 months, within 8 months, within 10 months, within 12 months, within 14 months, within 16 months, within 18 months, within 20 months, within 22 months, within 24 months, etc. e.g. within 3 years.

A similar procedure can be used to select markers of diagnostic value, e.g. by comparing autoantibody amounts of UC patients with healthy subjects, preferably healthy subjects that did not develop US in a given time frame as described above.

Marker comparison to determine diagnostic markers can be a fold change or AUC value estimation; preferably with an increase or decrease in fold change by e.g. at least 1.5 may indicate significance of the marker. For AUCs, values above 0.6 may indicate significance.

The inventive markers are given in tables 3, 4, 5, 6, 7, 8, 9, 10, 11, 12, 13, 14, 15 and 16. Any one of these markers alone, in combination with any marker(s) of any table or in combination with any marker(s) of the same table can be used. Markers are given by gene names or gene symbols. Full names and further information is known in the art and can be easily consulted in suitable databases, such as GeneCards (www.genecards.org) by using the indicated gene symbol. Other databases are available at EBI (www.ebi.ac.uk), NCBI (www.ncbi.nlm.nih.gov) or by the UniProt (www.uniprot.org) databases (date as of filing date of this application). Although the detection of a single marker/antigen/autoantibody of the markers of tables 3-16 can be sufficient to indicate a risk for UC or diagnose/detect UC, it is preferred to use more than one antigen, e.g. 2, 3, 4, 5, 6, 7, 8, 9, 10, 11 or 12 or more antigens/autoantibodies in combination, especially as said above, e.g. for simultaneous determination.

Preferably, the autoantibody is not ANCA (Antineutrophil cytoplasmic antibody). ANCA includes cytoplasmic ANCA (cANCA) and perinuclear ANCA (pANCA), both are preferably excluded. ANCA is found in Crohn'S Disease (CD) and is preferably excluded to avoid confusion between colitis ulcerosa (UC) and CD. Preferably, the autoantibody is not PAB (Pancreatic antibody). PAB is also found in Crohn's Disease and furthermore shows irregular patterns based on subject's race or lifestyle and is unsuitable for (UC) diagnosis (Mitsuyama et al., *supra).* ANCA and PAB are the only autoantibodies investigated so far and both are not suitable for UC diagnosis (Mitsuyama, *supra).* They have not been investigated for a predisposition analysis.

In especially preferred embodiments, the antigen(s) is/are selected from antigens of the group consisting of Table 16 and DCAF5 (DCAF5, RNASEK, CD58, VWF, CDCA4, RNF10, EEF1A1, DRG1, MINK1, CAPZA2, CPNE1, EIF2S3, FADS1, EVL, DHX8, EIF4A2, LAMTOR1, HNRNPA2B1, SIRT6, KIAA1731, FMNL2, GNAI2, FAM209B, TTC19, SRSF2, PHF17, CKB, MSL1, RUNDC3A, FAM13A, BCAR1, GOT1, KLC4, USP20, AP2S1, GALK2, PRDM8, WDR77, ALKBH2, R3HDM1, RPL36A, FAM131A, ZNF84, DYNC1I2, SUGP2, CCT3, EIF3G, TIAL1, USP28, SERPINF1, DST, LMO2, CENPT, HSP90AB1, C17orf62, NARS2, PPID, PLXNB2, WNK2, FTSJD2, PDHA1, GTF2I, PTCHD2, DPYSL3, TCTN3, UBE2L3, ZCCHC11, CHD3, CD81, TMED3, CHMP4A, SH3BGRL3, UBXN4, AAAS, FMNL1, CKAP5, DCX, CD99). In further embodiments, the antigens are selected from two or at least three antigens of the group of table 16 and DCAF5. Especially preferred antigen groups are HNRNPA2B1 (Heterogeneous Nuclear Ribonucleoprotein A2/B1), EVL (Enah/Vasp-Like), DCAF5 (DDB1 and CUL4 Associated Factor 5). These are most preferred for UC risk estimation. Another strongly preferred group is KLC4 (Kinesin Light Chain 4), PTCHD2 (Patched Domain-Containing Protein 2; also known as DISP3, Dispatched RND Transporter Family Member 3), KIAA1731 (also known as CEP295, Centrosomal Protein 295). These are most preferred for UC diagnosis of already present UC.

Of course any antigens (single or combinations, e.g. 2, 3 or more) selected from antigens of the group consisting of combined Tables 3-16 can be used. The markers are shown in the examples; in particular, they include the following markers as listed.
**Table 3:** DMPK, GGA1, HES1, RPS29, CD99, TRIM27, MCM3AP, POGZ, SLC16A8, FLAD1, NBPF15, SCAP, CD99, YES1, RPS17/ RPS17L, UBXN4, AP2S1, UXT, C9orf142, NACAD, DDR1;
**Table 4:** CD99, CAMKV, POGZ, GBF1, C17orf28, ADNP, NELL2, CHD3, TMEM98, GRM3, PODXL2, ATP6V1E1, HNRPLL, EIF4G3, HNRNPK, PIPSL, C11orf68, PAIP1, LRRC4B, ARF5, TBC1D1, PYGB, UBC, USP20, SGSM3, STIP1, STMN4;
**Table 5:** DMPK, HES1, RPS29, CD99, SLC16A8, AP2S1, UXT, FLAD1, POGZ, RPS17, RPS17L, COMT, METTL3, FGB, AHSG, PHF17, GBF1;
**Table 6:** DCAF13, C17orf70, TPP2, VARS2, RAB5C, DHX30, PTPN6, MI-NA, CNTD1, MCM6, ZWINT, GOLGA2, CLTA, CCDC94, HSF1, CCT2, FLNA;
**Table 7:** DCAF5, LAMC1, SULT1A3, SULT1A4, EVL, CKAP5, TINF2, CD58, PTCHD2, PTPRE, EIF2S3, TIAL1, FAM13A, USP11, FMNL2, HNRN-PA2B1, BCAR1, CPNE1, EEF1A1, FADS1, MAGED2, PPID, SUGP2;
**Table 8:** PTCHD2, USP11, HNRNPA2B1, CPNE1, EEF1A1, GNAI2, HNRPDL, DRG1, PKM, CAPZA2, ESYT1, ACD, GPSM1, ELAVL3, FAM131A, AKR1B1, LMO4, RNPS1, ZNF132, AP2S1, BTBD6, SRSF2, PA2G4, YWHAH, CENPT, EIF2B4, KIAA1731, PMM1, VBP1, CCT3, RAP1GAP, ACTR1B, BCL11A, EEF1G, EIF4A2, MPST, MRPL28, AURKAIP1, PPP6R2, PRKAR1B, PRMT1, TUBA1B, TMSB10/TMSB4X, USP20, ARPP19, CCT7, HNRNPK, NRXN2, TBRG4, APEX1, FAM65A, MICAL1, NUDCD3, PNMA1, SPINT2, UBA1, ULK1, C11orf2, CSDC2, DENND5A, AP1B1, ATAT1, BZW2, RBM5, TERF2IP, BCKDHA, PLXNB1, SRI, ADAMTS10, ENO1, SMARCC2, SPTAN1, SRA1, ZC3H11A, CLIC1, NDN, PFKM, POR, PRDX1, RPL15, PJA1, RBM10, AES, AKR1C4, COPZ1, HSD17B10, NACA, NYNRIN, PCID2, PTPRA, RNF216, TMEM132A, WBP2, ARPC1A, EIF3C/EIF3CL, GTF2IRD1, HINT1, NAE1, RPL19, VIM, CTC1, DDX39B, PLCB3, CALM1, DLGAP4, GRK6, GTF2B, RRP1, TPI1P2, PLAUR, TNC, CSNK2B, EPN1, NELL2, PPFIA1, SCAF1, SNX17, ZNF513, PASK, DNAJB1, EIF4G2, EXOSC8, FAM193B, RASGRP2, HUWE1, SNRNP70;
**Table 9:** DCAF5, PPID, GBAS, LRP5, PKM, WNK2, POGLUT1, AAAS, LMO4, AP2S1, SLC22A17;
**Table 10:** EVL, HNRNPA2B1, CD58, EEF1A1, DRG1, DHX8, GNAI2, ALKBH2, BCAR1, TIAL1, FADS1, EIF2S3, PTCHD2, LAMTOR1, RNF10, FAM209B, CDCA4, FAM13A, SUGP2, CPNE1, VWF, GOT1, ZNF84, RUNDC3A, FMNL2, RNASEK, C17orf62, FAM131A, MINK1, SH3BGRL3, SIRT6, CKB, PLXNB2, CCT3, UBE2L3, USP28, TTC19, EIF4A2, FTSJD2, R3HDM1, PPID, WNK2, HSP90AB1, PDHA1, KIAA1731, RPL36A, DST, CAPZA2, EIF3G, SRSF2, DYNC1I2, NARS2, ZCCHC11, GTF2I, MSL1, AAAS;
**Table 11:** COA5, YES1, MCM3AP, RANBP1, CKAP5, FMNL1, MATR3, KHDRBS1, TRAPPC4, TMEM59L, CCT2, PSMC2, SEC13, NARFL, DBN1, A1BG, ARF5, NARS2, GBA2, ZBTB45, BZRAP1, EPB42, ME3, SERPINF1, HID1 (also known as C17orf28), ANK2, TINAGL1, UBXN4, XRCC6, FLAD1, TRIM27, METTL3, NDUFS7, NDRG2, SULT1A3/SULT1A4, HMGA1, CDK9, KIAA1731, PSMD8, RPA3, KIAA0195, STT3A, DENND5A, RAB14, PTCHD2, DST, HSP90AB1, HSP90AB1, VPS35, DTX1, EIF4G3, SORBS2;
**Table 12:** CKAP5, FMNL1, CCT2, GBA2, ME3, SERPINF1, MAGED2, ANK2, FLAD1, METTL3, SULT1A3/SULT1A4, , KIAA1731, RPA3, ZNF622, PRDM8, EPS15L1, GNAS, ADK, PRRT2, C17orf108, SOX11, RHOT2, RPS29, DPYSL3, SNRNP25, C20orf27, AP2M1, TBC1D1, HES6, ENOX1, TCTN3, PSMB1, LCMT1;
**Table 13:** DCAF5, CKAP5, PTCHD2, USP11, FMNL2, C17orf62, MINK1, RUNDC3A, ALKBH2, KLC4, RANBP1, DPYSL3, CKB, CAPZA2, R3HDM1, CAPZA2, FAM131A, HSP90AB1, AP2S1, DTX1, DST, COA5, PA2G4, CHMP4A, RBM22;
**Table 14:** CD58, SUGP2, BCAR1, PTCHD2, GOT1, DRG1, FADS1, HNRN-PA2B1, FMNL2, USP28, EIF2S3, ALKBH2, SH3BGRL3, TTC19, C17orf62, GNAI2, RUNDC3A, DHX8, TIAL1, RNASEK, FAM13A, EVL, SIRT6, MAPT, UBE2L3, ZNF84, MINK1, CKB, CDCA4, CCT3, VWF, FAM209B, RNF10, FTSJD2, CPNE1, LAMTOR1, PLXNB2, EEF1A1, EIF4A2, FAM131A, MAGED2, BTBD6, STMN3, USP11, GBAS, ESYT1, PTPRE, CAPZA2, PHC2, ALDH1B1, RBM11, COMMD7, TRO, SULT1A3/SULT1A4, RANBP1, CEP57, CLN5, MLST8, NEUROD2, DCAF5, YWHAH, RAB34, RFC2, RELA, KLC4, DNTTIP2, ACD, CRYM, ZNF132, LOC494127, GSTM3, GPSM1, LAMC1, ZNF362, AGXT2L2, TINF2;
**Table 15:** KLC4, PTCHD2, KIAA1731, DST, DPYSL3, PRDM8, NARS2, USP20, CD81, CENPT, WDR77, UBXN4, FMNL1, AP2S1, CHMP4A, TCTN3, CKAP5, PHF17, GALK2, LMO2, SERPINF1, DCX, CHD3, CD99, TMED3;
**Table 16:** all markers of tables 10 and 15;
Some markers are connected by a "/" sign. This indicates a pair of equivalent markers. Any one or both of them can be used as alone or in combination with further markers.

In particular embodiments, the invention provides the method of diagnosing colitis ulcerosa or the risk thereof in a subject by detecting at least 2, 3, 4, 5, 6 or more or any number as disclosed above, of the marker proteins selected from the markers of each Table 3-16 combined in a subject comprising the step of detecting (auto)antibodies binding said marker proteins in a sample of the subject. Also provided is a method of diagnosing colitis ulcerosa or the risk of colitis ulcerosa in a subject by detecting at least 20%, preferably at least 30%, especially preferred at least 40%, at least 50%, at least 60%, at least 70%, at least 80% at least 90% or all of the markers selected from the markers of each Table 3-16 combined in a subject comprising the step of detecting (auto)antibodies binding said marker proteins in a sample of the subject. Such selections of markers are also referred to as "subsets" herein.

Preferred subsets of markers that are used in combination with one another are marker groups of the 2 top ranked or 3 top ranked markers of the Tables. "top ranked" means listed first in the tables, i.e. they have the highest characteristic or diagnostic value as determined in the table, such as fold change or AUC. Preferred subsets are: of Table 3: DMPK, GGA1, preferably further HES1; of Table 4: CD99, CAMKV, preferably further POGZ; of Table 5: DMPK, HES1, preferably further RPS29; of Table 6: DCAF13, C17orf70, preferably further TPP2; of Table 7: DCAF5, LAMC1, preferably further SULT1A3; of Table 8: PTCHD2, USP11, preferably further HNRNPA2B1; of Table 9: DCAF5, PPID, preferably further GBAS; of Table 10: EVL, HNRNPA2B1, preferably further CD58; of Table 11: COA5, YES1, preferably further MCM3AP; of Table 12: CKAP5, FMNL1, preferably further CCT2; of Table 13: DCAF5, CKAP5, preferably further PTCHD2; of Table 14: CD58, SUGP2, preferably further BCAR1; of Table 15: KLC4, PTCHD2, preferably further KIAA1731.

The markers of Tables 3-16 have been grouped in said tables because within each Table, the markers work well together and synergistically improve a diagnosis (including risk determination) if the markers are selected from the markers within respective table. Accordingly, preferably the invention provides the method of diagnosing colitis ulcerosa or the risk thereof in a subject by detecting at least 2, 3, 4, 5, 6 or more or any number as disclosed above, of the marker proteins selected from the markers of any table selected from Tables 3-16 in a subject comprising the step of detecting (auto)antibodies binding said marker proteins in a sample of the subject. Also provided is a method of diagnosing colitis ulcerosa or the risk of colitis ulcerosa in a subject by detecting at least 20%, preferably at least 30%, especially preferred at least 40%, at least 50%, at least 60%, at least 70%, at least 80% at least 90% or all of the markers selected from the markers of any table selected from Tables 3-16 in a subject comprising the step of detecting (auto)antibodies binding said marker proteins in a sample of the subject. Especially preferred is table 10. Preferably any number of these markers of table 10 are selected, in particular preferred in the order of ranking according to fold change, e.g. the most 2, 3, 4, 5, 6, 7, 8 or more markers with the highest fold change. Of course these numbers in the order as listed can be applied to the selection of markers from any table 3-16.

The markers of Tables 7-16 are particularly optimised for risk of UC determination. Accordingly, the invention provides a method wherein the risk of developing colitis ulcerosa is determined and the antigens are selected from any one of tables 7, 8, 9, 10, 11, 12, 13, 14, 15, 16 or of combined tables 7-16.

The markers of Tables 3-6 are particularly optimised to diagnose an existing UC condition. Accordingly, the invention provides a method wherein colitis ulcerosa is diagnosed and the antigens are selected from any one of tables 3, 4, 5, 6 or of combined tables 3-6. It is possible to determine the severity of colitis ulcerosa by comparing with autoantibody amounts in the sample against the antigens of table 6. Such a severity can be estimated according to the CAS. The CAS (abbreviation of "Clinical Activity Score") is known and used in the art, e.g. as disclosed by Walmsley et al. Gut 1998, 43:29-32, and Walsh et al., Gastroenterol Hepatol (N Y). 2012; 8(11): 751-754, both incorporated herein by reference)

In the inventive method of diagnosis, including the risk determination, a comparison with known comparative values for autoantibody determination against the inventive markers can be performed. Comparative values can be from a healthy subject, a UC patient or a subject that developed UC but was UC free at the point of autoantibody collection (the collected autoantibodies are determined). The latter can be used for risk evaluation. The UC free time can be as described above, e.g. 12 months etc.. The inventive method may comprise the step of detecting antibodies binding said antigens and comparing said detection signal with detection signals of a healthy control and comparing said detection signals, wherein an increase in the detection signal indicates colitis ulcerosa or a risk thereof, except for antibodies against antigens RASGRP2, HUWE1, SNRNP70, STMN4, wherefore a decrease indicates colitis ulcerosa or a risk thereof. Alternatively or in addition the step of detecting antibodies binding said antigens may comprise comparing said detection signal with detection signals of one or more known colitis ulcerosa control sample(s). The control signals may be used to obtain an antigen dependent signal pattern as indication classifier. Then the antigen dependent signals of the subject is compared with said pattern and/or fitted onto said pattern, thereby obtaining information of the diagnosed condition. The method may further comprise comparing said detection signals. A detection signal from the sample of the subject in amplitude (or level, amount concentration,- according to the value that is determined by the method) of at least 60%, preferably at least 80%, of the colitis ulcerosa control may indicate colitis ulcerosa. Alternatively, a detection signal in at least 60%, preferably at least 75%, of the used antigens/markers may indicate colitis ulcerosa or the risk thereof. In case of a comparison with a healthy control, an increase by at least 10%, preferably at least 20%, more preferred by at least 30%, may indicated colitis ulcerosa or a risk thereof - except in cases of markers RASGRP2, HUWE1, SNRNP70, STMN4, wherein a decrease by at least 10%, preferably at least 20%, more preferred by at least 30%, may indicate colitis ulcerosa or a risk thereof. These are just examples to achieve a good diagnosis or risk estimation. Any comparison as commonly known in the art may be used. It is also possible to obtain fresh comparison values with controls before the diagnosis or risk estimation for UC before testing the sample of the subject.

Colitis ulcerosa or a predisposition or risk of developing UC as diagnosed by the inventive method may be treated. A treatment of UC may relate to the administration of anti-inflammatory compounds (Burger et al., Viszeralmedizin 2015;31:236-245, incorporated herein by reference). Accordingly, the invention also related to a method of treating a subject having colitis ulcerosa or being at risk of developing colitis ulcerosa, comprising detecting colitis ulcerosa or the risk thereof according to the invention and administering a colitis ulcerosa medication, preferably an anti-inflammatory medicament. A treatment of a subject diagnosed with UC according to the invention or being at risk thereof according to the inventive risk estimate, may comprise administration of anti-UC pharmaceuticals such as mesalazine (mesalamine or 5-aminosalicylic acid), a TNF-alpha antagonist, such as an anti-TNF-alpha antibody, e.g. infliximab (Remicade^{®}), adalimumab or golimumab, an Nonsteroidal Anti-Inflammatory Drug (NSAID), a steroidal anti-inflammatory drug, a budesonide, salicylic acid, azathioprine, acetylsalicylic acid, a corticosteroid, e.g. prednisone, or a calcineurin inhibitor like cyclosporine or tacrolimus, an integrin antagonist like anti-integrin antibodies, e.g. vedolizumab, etrolizumab or natalizumab, a JAK inhibitor like tofacitinib, an IL13 inhibitor, like an anti-IL13 antibody like tralokinumab, anrunkinzumab, BMS-936557, HMPL-004, DIMS0150, a toll-like receptor agonist, in particular a TLR-9 agonist like DIMS0150, PF-00547659, RPC1063, heparin, or combinations thereof (see Burger et al., supra). The inventive treatment does not claim a capacity to fully heal UC. Instead the inventive treatment relates to an amelioration of symptoms and/or a reduction of molecular-mechanistical causes of UC. Which treatment may be suitable for a given patient may depend on the severity of UC and tolerability of a particular treatment.

The invention also provides such a pharmaceutical or medicament for use in the treatment or prevention (preventive treatment) of UC that is diagnosed by the invention or its risk has been estimated by the invention. Also provided is a method of manufacturing a medicament for the treatment of UC or prevention of UC (as above) comprising diagnosing a subject with UC according to the invention or estimating the risk of UC in a subject according to the invention. The medicament may comprise any one of the above pharmaceuticals.

In a further aspect, the invention relates to a kit of diagnostic agents suitable to detect autoantibodies against any antigen or antigen combination as defined above and in the examples, especially tables 3-6 and 7-16, wherein said diagnostic agents comprise antigens suitable to bind autoantibodies in a sample. In some embodiments, said diagnostic agents, e.g. the antigens are immobilized on a solid support. In combinable or alternative embodiments, the diagnostic agents, e.g. the marker antigens or secondary antibodies are labelled by a nucleotide tag, such as e.g. for immuno-PCR. Preferably the diagnostic agents are immobilized on a solid support or present or reconstitutable in solution, especially when said markers are each labelled with a unique label, such as a unique nucleic acid sequence tag. The inventive kit may further comprise detection agents, such as secondary antibodies, in particular anti-human antibodies, and optionally also buffers and dilution reagents. The invention therefore likewise relates to the object of providing a diagnostic device or an assay, in particular a protein biochip, ELISA or Immuno-PCR assay, which permits a diagnosis or examination for colitis ulcerosa.

Additionally, the marker proteins (as binding moieties for antibody detection) can be present in the respective form of a fusion protein, which contains, for example, at least one affinity epitope or tag. The tag may be one such as contains c-myc, his tag, arg tag, FLAG, alkaline phosphatase, VS tag, T7 tag or strep tag, HAT tag, NusA, S tag, SBP tag, thioredoxin, DsbA, a fusion protein, preferably a cellulose-binding domain, green fluorescent protein, maltose-binding protein, calmodulin-binding protein, glutathione S-transferase, or lacZ, a nanoparticle or a nucleic acid sequence tag. Such a nucleic acid sequence can be e.g. DNA or RNA, preferably DNA.

As said above, antigens may be complete of full-length proteins. The antigens may also be proteins or peptides that contain an epitope that is bound by the autoantibodies. Any such antigen can be used in the inventive kit.

In all of the embodiments, the term "solid support" covers embodiments such as a filter, a membrane, a magnetic or fluorophore-labeled bead, a silica wafer, glass, metal, ceramics, plastics, a chip, a target for mass spectrometry, a matrix, a bead or microtiter well. However, a expoxy-coated glass(microarray slides) is preferred according to the invention.

As a filter, furthermore PVDF, nitrocellulose, or nylon is preferred (e.g., Immobilon P Millipore, Protran Whatman, Hybond N+ Amersham).

In another preferred embodiment of the arrangement according to the invention, the arrangement corresponds to a grid with the dimensions of a microarray e.g in a microscope slide format or microtiter plate (8-12 wells strips, 96 wells, 384 wells, or more), a silica wafer, a chip, a target for mass spectrometry, or a matrix.

Another method for detection of the markers is an immunosorbent assay, such as ELISA. When detecting autoantibodies, preferably the marker protein or at least an epitope containing fragment thereof, is bound to a solid support, e.g. a microtiter well. The autoantibody of a sample is bound to this antigen or fragment. Bound autoantibodies can be detected by secondary antibodies with a detectable label, e.g. a fluorescence label. The label is then used to generate a signal in dependence of binding to the autoantibodies. The secondary antibody may be an anti-human antibody if the patient is human or be directed against any other organism in dependence of the patient sample to be analysed. The kit may comprise means for such an assay, such as the solid support and preferably also the secondary antibody. Preferably the secondary antibody binds to the Fc part of the (auto) antibodies of the subject. Also possible is the addition of buffers and washing or rinsing solutions. The solid support may be coated with a blocking compound to avoid unspecific binding.

Preferably the inventive kit also comprises non-diagnostic control proteins, which can be used for signal normalization. These control proteins bind to moieties, e.g. proteins or antibodies, in the sample of a UC or UC risk subject as controls. In addition to the inventive marker proteins any number, but preferably at least 2 controls can be used in the method or in the kit.

The kit may optionally further comprise a computer-readable medium or a computer program product, such as a computer readable memory devices like a flash storage, CD-, DVD- or BR-disc or a hard drive, comprising signal data for control samples with known conditions selected from colitis ulcerosa or risk of colitis ulcerosa, and/or calibration or training data for analysing said antigens provided in the kit for diagnosing colitis ulcerosa or risk of colitis ulcerosa or distinguishing conditions selected from healthy conditions, colitis ulcerosa and risk of colitis ulcerosa. Alternatively or in addition, the kit may comprise calibration standards or cut-off-values based on autoantibody amount (IgG- or sample-amount used for analysis).

The kit may also comprise instructions for performing any one of the inventive methods. Such instructions may be in the form of a leaflet or computer readable information.

Preferably the inventive kit is limited to a particular size. According to these embodiments of the invention the kit comprises at most 3000 diagnostic agents, preferably at most 2500 diagnostic agents, at most 2000 diagnostic agents, at most 1500 diagnostic agents, at most 1200 diagnostic agents, at most 1000 diagnostic agents, at most 800 diagnostic agents, at most 500 diagnostic agents, at most 300 diagnostic agents, at most 200 diagnostic agents, at most 100 diagnostic agents; and or further preferred wherein the kit comprises a labelled secondary antibody, preferably for detecting an Fc part of autoantibodies of the subject. The kit may also just comprise one diagnostic agent.

The kit may also comprise normalization standards, that result in a signal independent of a UC or UC risk. Such normalization standards can be used to obtain signals dependent on the amount of total IgG applied. Such standards may be specific for ubiquitous antibodies found in a human. Preferably the normalization standards include positive and negative (leading to no specific signal) normalization standards.

The invention also provides the method of manufacture of a kit, especially for use in the above described methods.

The present invention is further explained and illustrated by the following figures and examples, without necessarily being limited to these aspects of the invention.

### Figures:

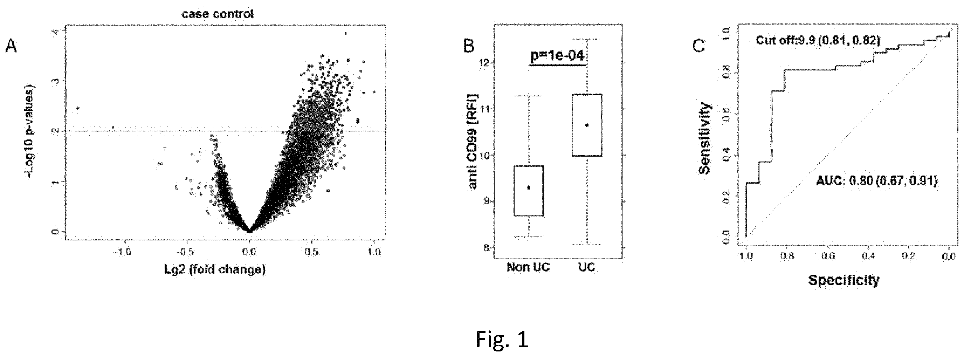

**Figure 1****: Autoantibodies as biomarkers to discriminate between UC and non-UC donors.** (A) Increased autoantibody reactivity in UC patients displayed as a volcano plot. Purified serum IgG of UC (49) and non-UC (23) patients were subjected to protein-microarray analysis of UC vs non-UC. Antigenic reactivity in cases versus controls is presented. Significantly differentially reactive antigens are given in blue (n=697; p<0.01; y-axis: "-log10 p value"; x-axis shows the log2 - fold change between classes). (B) Expression levels of anti-CD99 antibodies in UC (n=49) and non-UC (n=19) donors depicted as a boxplot diagram. RFI, Log2 of median relative fluorescent intensity. Boxes represent upper and lower quartiles and whiskers represent variability. For comparison of non-UC versus UC, a Student's t-test was performed. (C) ROC curve for anti-CD99 antibodies in differentiating UC and Non-UC patients. Sample sizes: non-UC n=19, UC n=49.

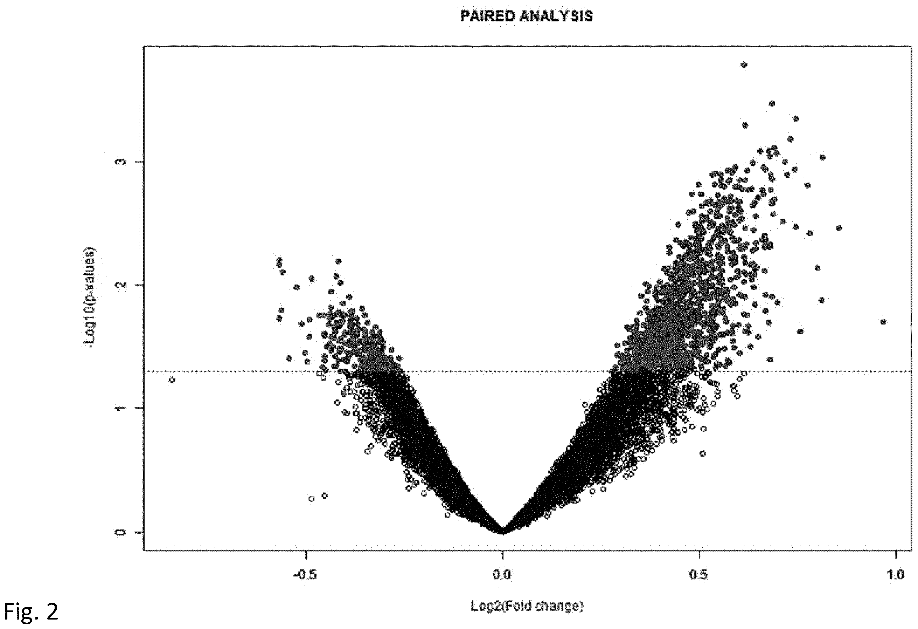

**Figure 2****: Volcano plot derived from paired analysis of T3 vs T-early samples.** The majority of 1201 features have higher reactivity in samples at T3 closest to clinical UC presentation; vice versa only 242 of 1452 have higher reactivity at T-early; although the threshold for significance was set to a moderate p<0.05 value, and thus a false positives might be relatively high, the overwhelming number of antigens higher reactive at time close to diagnosis is in line with the findings described above when severe UC (under therapy) versus controls showed a similar pattern, indicating breach of tolerance and high autoantibody reactivity versus self-antigens.

### Examples:

### Example 1: Patient samples and control:

"LMU" samples - samples comprise severe UC patients under treatment. Serum samples from patients (n=49) and controls (n=23; age and gender matched) were collected and stored frozen within about 4h upon blood collection at -80°C. Aliquots of serum were then used for IgG isolation (as described in Example 2). Patient characteristics are summarised in Table 1.

**Table 1a. LMU - patient samples: Baseline demographics, duration of disease and therapy of patients, serum samples have been used for antibody profiling.**

| | UC | Non-UC |
|---|---|---|
| | N=49 | N=23 |
| Age (years) | | |
| Mean (SD) | 38.5 (15.6) | 36.7 (15.9) |
| Range | 24-74 | 21-59 |
| Gender (% male) | 46 | 42 |
| Duration of UC (years) | | |
| Mean (SD) | 11.6 (9.53) | |
| Range | 1-40 | |
| SCCAI | | |
| Mean (SD) | 3.04 (2.79) | |
| Range | 0-13 | |
| Treatment (current) | | |
| TNFα-blocker | 20 | |
| Glucocorticoids | 13 | |
| Mesalazine | 26 | |
| Immuno-suppressive | 6 | |
| No | 10 | |

"CAS" means Clinical Activity Score as introduced by Walmsley et al. Gut 1998, 43:29-32, which his used to assess disease activity in UC (Walsh et al., Gastroenterol Hepatol (N Y). 2012; 8(11): 751-754). The CAS CLINICAL Activity Score of LMU-patients is summarized in table 1b.

**Table 1b: CAS - Clinical Activity Score of LMU-patients**

| CAS score | LMU-patients (49 in total) |
|---|---|
| 0 | 10 |
| 1 | 10 |
| 2 | 9 |
| 3 | 6 |
| 4 | 3 |
| 5 | 5 |
| 6 | 2 |
| 7 | 1 |
| 9 | 1 |
| 10 | 1 |
| 13 | 1 |

"BRK" samples - blood bank samples of people that later developed UC but were disease free at the time of donation. BRK samples are a plasma sample cohort of 33 UC cases and 33 controls from the "Biobank, Bayrisches Rotes Kreuz" comprising samples of 11 individuals at three different time-points collected 1-65 month prior clinical diagnosis (see Example 7, Table 2).

### Example 2: Immunoglobulin (IgG) purification of blood samples

Immunoglobulin G purification was performed using MelonGel resins (Pierce) as described in (Rosskopf et al., J. Immunol. Methods, 2015;418:39-51). Briefly, purification of all samples was performed according to the manufacturer's instructions of the Melon^{™} Gel IgG Purification Spin Plate Kit (Thermo Scientific, Waltham, MA, USA) using 15 µl of plasma, followed by determination of IgG concentration as described in (Rosskopf et al., *supra*). Sample integrity was determined by running each purified sample on a NuPAGE® Novex 4-12% Bis-Tris Precast Gel (Life Technologies, Carlsbad, CA, USA).

### Example 3: Protein microarray production and processing

Protein expression, purification and microarray production was performed as described in (Brezina et al., Microarrays 2015, 4:162-187). Briefly, human in frame cDNA protein expression library consisting of 15,417 *E. coli* cDNA expression clones presenting 6,369 unique, distinct human proteins were expressed and His-tagged recombinant proteins were purified and eluted. Concentration standardized proteins were spotted in duplicates using an Omnigrid arrayer on ARChip Epoxy glass slides (Preininger et al., 2004, Anal. Biochem. 330, 29-36.). Protein microarray processing was done as previously described (Brezina et al., 2015, *supra*).

Due to array processing handling capacity, arrays were processed on 2 different batches of protein arrays for LMA (UC) and BRK (UC risk) samples, in different runs (run1: 49 CRC vs 23 controls; BRK: 11 Cases x 3 time points before UC clinical diagnosis and 33 controls matched with respect to time points, date and blood collection center. All these BRK samples were collected from blood donations, conducted by the Bavarian Red Cross. The control samples have been selected from the BRK biobank and matched to case-samples for the collection date using control samples obtaining the blood donation the same day in the specific center as cases.

### Example 4: Data acquisition and statistical analysis

In order to scan array images and to align and grid the predefined features, the GenePix Pro Microarray Acquisition & Analysis Software 6.0 of Molecular Devices (Sunnyvale, CA, USA) was used.

Data normalization, and statistical data analysis was performed on microarray data using R 3.0.1 and BRB-ArrayTools (V. 4.3.1) developed by Dr. R Simon and Amy Lam (Simon et al., 2007, Cancer Inform. 3, 11-17). Correction for the systematic bias that may have been introduced by protein array printing (e.g. pin-filling, or batch effects) was conducted by identification of systematic effects by NMF and/or k-means clustering, then assigning cluster information to each data. Class comparison analyzes between the groups of arrays were performed by computing a t-test, and defining proteins differentially expressed among the classes of samples; when appropriate class comparison analysis was conducted applying blocking effects. Differentially immune-reactive proteins /antigens (DIRAG) between different sample classes were depicted using a p-value as specified in results. DIRAGs ID annotations with its ratio of geometric mean (fold-change) between sample groups and p-values are then further considered for specifying candidate antigenic markers. Top tables based on p-values and fold-changes obtained from different analysis are then compiled and intersected from severe UC (LMU) vs controls and pre-diagnosis UC patients vs matched controls (BRK samples).

### Example 5: Autoantibody profiling of severe UC vs controls serum-IgG samples

We have conducted antibody profiling on AIT's 16k protein array using 49 UC samples and 23 controls (includes 3 samples specified after having conducted analysis as "other autoimmune disease"). Upon purification from serum samples, IgG concentration measured, we have found a statistically significant difference (p=0.0027) of IgG amounts / concentrations in serum from UC cases (median=10.64 mg/ml serum) vs controls (8.86mg/ml). Although when loading a standardized IgG amount of 67.5 µg onto microarrays, class comparison analysis between cases and controls elucidated 697 significant DIRAGs at the nominal p<0.01 level. Sorting the top list derived by the fold-change between classes, elucidates with higher antigenic reactivities in cases for proteins like DMPK, GGA, HES, RPS29, CD99 etc; of these CD99 presented by multiple clones on the arrays is reproducibly listed (Table 3, Fig. 1).

**Table 3: Top antigens (derived from 697 significant; p<0,01) ranked by fold change**

| **Parametric p-value** | **FDR** | **Geom mean of intensities in class 1** | **Geom mean of intensities in class 2** | **Fold-change , increase** | **Name** |
|---|---|---|---|---|---|
| 0.0016529 | 0.0795 | 1282.1 | 640.82 | 2 | DMPK |
| 0.001753 | 0.0795 | 1818.41 | 962.46 | 1.89 | GGA1 |
| 0.0004156 | 0.0795 | 1788.2 | 947.7 | 1.89 | HES1 |
| 0.0006475 | 0.0795 | 1283.67 | 690.63 | 1.86 | RPS29 |
| 0.0008848 | 0.0795 | 1485.46 | 805.73 | 1.84 | CD99 |
| 0.005878 | 0.0796 | 937.25 | 513.29 | 1.83 | TRIM27 |
| 0.0065022 | 0.0825 | 2056.95 | 1124.54 | 1.83 | MCM3AP |
| 0.0013648 | 0.0795 | 2432.38 | 1374.53 | 1.77 | POGZ |
| 0.0015698 | 0.0795 | 2069.3 | 1180.18 | 1.75 | SLC16A8 |
| 0.0018793 | 0.0795 | 856.75 | 490.21 | 1.75 | FLAD1 |
| 0.0003951 | 0.0795 | 354.27 | 203.84 | 1.74 | NBPF15 |
| 0.001114 | 0.0795 | 467.47 | 267.9 | 1.74 | SCAP |
| 0.0036918 | 0.0795 | 2389.08 | 1371.05 | 1.74 | CD99 |
| 0.0034252 | 0.0795 | 512.57 | 295.68 | 1.73 | YES1 |
| 0.0012119 | 0.0795 | 1889.77 | 1089.48 | 1.73 | RPS17/RPS17L |
| 0.0023996 | 0.0795 | 810.49 | 469.71 | 1.73 | UBXN4 |
| 0.0008638 | 0.0795 | 1072.27 | 622.45 | 1.72 | AP2S1 |
| 0.0009088 | 0.0795 | 3789.48 | 2216.4 | 1.71 | UXT |
| 0.0001134 | 0.0795 | 567.53 | 331.13 | 1.71 | C9orf142 |
| 0.0038452 | 0.0795 | 555.97 | 327.83 | 1.7 | NACAD |
| 0.0007992 | 0.0795 | 503.34 | 295.95 | 1.7 | DDR1 |

Applying more stringent filtering criteria on data (using the option in BRBAT to "Exclude a protein under mean intensity value of 500; using averaged spots & median normalized data) elucidates 481 significant antigens between cases and controls (p<0.01; & fold change 1.35 between groups).

Of these, 27 antigenic proteins were listed ≥2 times in this list of 481 significant features when expressed from different clones and presented by distinct duplicate spots on arrays. This allows the use of these markers in diagnostics (Table 4 - 27-multiple features):

**Table 4:**

| Of 481 there were 27 antigenic proteins presented by multiple features / hits on arrays (≥2): | | |
|---|---|---|
| NAME | hits significant | mean - fold-change, increase except STMN4 |
| CD99 | 2 | 1.79 |
| CAMKV | 2 | 1.675 |
| POGZ | 2 | 1.665 |
| GBF1 | 2 | 1.64 |
| C17orf28 | 2 | 1.605 |
| ADNP | 2 | 1.59 |
| NELL2 | 2 | 1.57 |
| CHD3 | 4 | 1.525 |
| TMEM98 | 2 | 1.525 |
| GRM3 | 2 | 1.52 |
| PODXL2 | 3 | 1.51 |
| ATP6V1E1 | 2 | 1.505 |
| HNRPLL | 2 | 1.5 |
| EIF4G3 | 2 | 1.495 |
| HNRNPK | 2 | 1.49 |
| PIPSL | 3 | 1.487 |
| C11orf68 | 2 | 1.48 |
| PAIP1 | 2 | 1.48 |
| LRRC4B | 2 | 1.475 |
| ARF5 | 2 | 1.47 |
| TBC1D1 | 2 | 1.46 |
| PYGB | 2 | 1.455 |
| UBC | 2 | 1.445 |
| USP20 | 2 | 1.445 |
| SGSM3 | 2 | 1.405 |
| STIP1 | 2 | 1.38 |
| STMN4 | 2 | 0.425 (decrease) |

Unsupervised clustering revealed stable clusters when using k-means clustering for 4 clusters. These elucidates high significantly different array-/ sample-groups derived from confounding effects, although sampling and array processing was highly standardized. When considering single spots of different duplicate spotted proteins and conducting class comparison for these 4 clusters, 6792 features (of 8902 pre-filtered for intensity from entire number of 32640 features on arrays are delineated (p< 1e-05; data not shown).

Cluster 1 presented 18 cases and 11 controls, cluster 2: 13 cases and 4 controls, cluster 3: 10 cases and 7 controls, cluster 4: 8 cases and 1 control. Then considering these different clusters (but excluding cluster 4 from analysis in which only 1 control but 8 cases were present) for elucidation of DIRAGs between cases vs control we conducted class comparison blocking analysis by the cluster elucidating 352 proteins differentially reactive (using a cut-off: p<0.01 and 1.35 fold change between classes).

Ranking significant proteins by the fold change (case vs controls ≥1.6), elucidates a list of 16 proteins (with DMPK, HES1, RPS29 and CD99, SLC16A8 on top (Table 5). Comparing the 352 significant antigens with the 27 antigenic proteins listed ≥2 times in top-list of the primary analysis (according Table 4), all but 4 antigens (C17orf28, ADNP, TMEM98, ATP6V1E1) are again presented, confirming reliability of findings.

**Table 5: DIRAG highest reactive in cases (class 1) vs controls (class 2) (sorted and filtered by fold change >1.6; p<0.01) derived from class comparison analysis blocked by k-means clusters (according Supplemental Tab S2).**

| **Parametric p-value** | **FDR** | **Geom mean of intensities in class 1** | **Geom mean of intensities in class 2** | **Fold-change ↑ (increase)** | **Name** |
|---|---|---|---|---|---|
| 0.0079021 | 0.445 | 1146.33 | 610.84 | 1.88 | DMPK |
| 0.001861 | 0.445 | 1752.53 | 934.91 | 1.87 | HES1 |
| 0.0010196 | 0.445 | 1176.11 | 658.43 | 1.79 | RPS29 |
| 0.0033467 | 0.445 | 1346.24 | 763.58 | 1.76 | CD99 |
| 0.0027352 | 0.445 | 1936.5 | 1115.16 | 1.74 | SLC16A8 |
| 0.0010419 | 0.445 | 1008.73 | 603.31 | 1.67 | AP2S1 |
| 0.0031616 | 0.445 | 3549.49 | 2128.69 | 1.67 | UXT |
| 0.0039713 | 0.445 | 813.7 | 488.12 | 1.67 | FLAD1 |
| 0.00377 | 0.445 | 2213.71 | 1342.13 | 1.65 | POGZ |
| 0.0038568 | 0.445 | 1495.95 | 909.61 | 1.64 | RPS17/RPS17L |
| 0.0064379 | 0.445 | 1132.93 | 694.56 | 1.63 | COMT |
| 0.0051155 | 0.445 | 1437.45 | 887.81 | 1.62 | METTL3 |
| 0.0012894 | 0.445 | 1846.21 | 1137.88 | 1.62 | FGB |
| 0.0025513 | 0.445 | 850.15 | 528.74 | 1.61 | AHSG |
| 0.0006666 | 0.445 | 982.85 | 614.36 | 1.6 | PHF17 |
| 0.0043943 | 0.445 | 1773.94 | 1108.66 | 1.6 | GBF1 |

### Example 6: further correlation with disease activity

Correlation analysis of antibody reactivities (fluorescent measures) on arrays with respect to clinical activity score (CAS - see example 1) elucidated 21 features (17 thereof specified and annotated by a gene-symbol). Thus disease activity is highly correlated (p<0.001 & correlation coefficients lower than - 0.455) with antibody reactivity (Table 6).

**Table 6: 17 antigens highly correlated with CAS score (p<0,001; Pearson Correlation Test; LMU-patients)**

| **#** | **Correlation coefficient ↓** | **Parametric p-value** | **Name** |
|---|---|---|---|
| 1 | -0,57 | 5,40E-05 | DCAF13 |
| 2 | -0,528 | 9,62E-05 | C17orf70 |
| 3 | -0,524 | 0,0001106 | TPP2 |
| 4 | -0,51 | 0,0001831 | VARS2 |
| 5 | -0,506 | 0,0002098 | RAB5C |
| 6 | -0,502 | 0,0002377 | DHX30 |
| 7 | -0,5 | 0,0002513 | PTPN6 |
| 8 | -0,487 | 0,0003836 | MINA |
| 9 | -0,477 | 0,0005248 | CNTD1 |
| 10 | -0,476 | 0,0005513 | MCM6 |
| 11 | -0,475 | 0,0005583 | ZWINT |
| 12 | -0,472 | 0,0006261 | GOLGA2 |
| 13 | -0,471 | 0,0006396 | CLTA |
| 15 | -0,464 | 0,0007823 | CCDC94 |
| 14 | -0,46 | 0,0008893 | HSF1 |
| 16 | -0,458 | 0,0009435 | CCT2 |
| 17 | -0,456 | 0,0009975 | FLNA |

Antigens with highest correlation DCAF13 and C17orf70 both involved in DNA damage repair and ubiquitinylation. C17orf70 - also known as FAAP100 is a component of the Fan-coni anemia (FA; MIM 277650) core complex and is required for core complex stability and FANCD2 (see MIM 227646). It is also functioning in monoubiquitination and induces chromosomal instability as well as hypersensitivity to DNA cross-linking agents, when repressed.

DCAF13 (DDB1 And CUL4 Associated Factor 13) related pathways are rRNA processing and Gene Expression, and GO annotations related to this gene includes poly(A) RNA binding. DDB1 (Damage Specific DNA Binding Protein 1) as described in the GeneCards data bank, like FAAP100 has a functional role in damage repair and ubiquitinylation. According to GeneCards information "Defective activity of the protein complex causes the repair defect in patients with xeroderma pigmentosum complementation group E (XPE; an autosomal recessive disorder characterized by photosensitivity and early onset of carcinomas. The protein encoded by this gene also functions as an adaptor molecule for the cullin 4 (CUL4) ubiquitin E3 ligase complex by facilitating the binding of substrates to this complex and the ubiquitination of proteins."

Thus autoantibody reactivity against these repair-complexes and associated proteins, impair biological function and are in line with known pathology. Therefore these findings corroborate elucidation of UC molecular pathology and provide mechanistically and biologically meaningful candidate-biomarkers, as well as assist defining novel therapy regimens by providing molecular therapeutic targets for improving UC management.

### Example 7: Pre-diagnostic UC plasma-samples:

In an independent experiment we analysed 66 samples comprising 33 samples from 11 UC patients derived from 3 different time-points prior diagnosis and 33 healthy controls matched for gender, the time and center of blood collection. Sample characteristics of these pre-diagnostic UC patients are summarized in Table 2.

**Table 2: UC-pre-diagnostic sample characteristics. Samples were obtained from the blood bank of the Bavarian Red Cross - biobank prior clinical UC diagnosis.**

| individuals | time (month) sample taken prior diagnosis | | | IgG concentration * [mg/ml plasma] | | | IgG increase (%) ** | delta T3-Tx (m) ** |
|---|---|---|---|---|---|---|---|---|
| | T1 | T2 | T3 | IgG T1 | IgG T2 | IgG T3 | | |
| 1 | 22 | **20** | **4** | 7,95 | **8,70** | **9,24** | 6% | 16 |
| 2 *** | **19** | 16 | **4** | **7,17** | 7,47 | **8,78** | 22% | 15 |
| 3 *** | **15** | 10 | **6** | **7,01** | 6,37 | **6,59** | -6% | 9 |
| 4 | **17** | 12 | **4** | **9,64** | 8,94 | **7,11** | -26% | 13 |
| 5 | 34 | **24** | **11** | 7,17 | **5,33** | **8,18** | 53% | 13 |
| 6 | 25 | **22** | **10** | 8,29 | **5,87** | **6,27** | 7% | 12 |
| 7 | 65 | **22** | **3** | 8,71 | **5,91** | **7,60** | 29% | 19 |
| 8 | **21** | 14 | **11** | **10,70** | 5,23 | **4,33** | -60% | 10 |
| 9 | **35** | 20 | **11** | **5,47** | 5,48 | **7,03** | 29% | 24 |

| | | | | | | | | |
|---|---|---|---|---|---|---|---|---|
| *IgG concentration measures upon MelonGel purification ** increase in IgG concentrations was evaluated for samples taken >12m (median 13m) before T3 closest to diagnosis - indicated by bold numbers in the time- and IgG concentration columns; *** case 2&3 provided poor classification, possibly due to age of sample | | | | | | | | |

Serum IgG concentrations in patients (considering T3 closest to DX; n=11) obtained a median IgG-serum concentration of 7.31 (SD=1.29) versus controls (median= 7.77 SD= 1.44; n=33) was not significantly different. Paired analysis of serum concentrations in UC at different time-points was then conducted considering 9 individuals when paired samples available within a year (4-11m) prior UC diagnosis and comparing that IgG serum concentrations with samples taken 9-24 (median 13 month before T3). In 9 patients - comparing IgG plasma concentrations at T3 versus samples taken 9-24 (median 13 month before T3), there is a 6,8% median increase in these 9 UC cases; however difference in serum-IgG between time-points is not significant (p=0.938; paired T-Test; Table 2).

Therefore we conclude that IgG plasma-concentrations in pre-diagnostic UC samples do not increase until diagnosis, but changes are affecting the reactivity profiles then targeting self-antigens.

Although a direct comparison of IgG concentrations from study groups (severe UC case /control *serum;* pre-diagnostic BRK case/control *plasma)* is not advisable due the systematic difference between serum vs plasma samples,- based on data described above, IgG concentrations might increase during the progression into severe as seen when *serum* IgG-concentrations were significantly higher in severe cases vs controls - whereas in pre-diagnostic study cohort the *plasma* IgG concentrations do not show significant changes in 1) cases vs controls as well as 2) in cases at time-points "closest to" vs " >2y before" UC diagnosis.

### Example 8: Auto-antibody increase prior clinical diagnosis - pre-diagnostic UC plasma

As indicated above, of the pre-diagnostic UC samples 9 individuals were selected for comparative analysis of a sample closest to clinical diagnosis (<12m) and a sample derived from an earlier time-point (median 13 month before T3 sample taken). When applying class comparison in a paired manner (single feature significance level of p<0.05), 1452 features (of 14983 features that passed filtering criteria) as illustrated in the volcano-plot (Figure 2).

Based on the fold change indicating higher auto-antigenic reactivity in samples closest to clinical UC diagnosis (time point T3 with a median 6m before clinical diagnosis) versus samples taken 13 month (median T-early) earlier, shows highest changes of FC >1.6 for 22 antigenic proteins which are listed in Table 7.

**Table 7: 22-antigenic proteins with a FC>1,6 derived from BRK sample testing**

| **Protein / gene symbol** | **Parametric p-value** | **FC (T3 vs Tearly)** |
|---|---|---|
| DCAF5 | 0,003 | 1,81 |
| LAMC1 | 0,013 | 1,75 |
| SULT1A3/SULT1A4 | 0,007 | 1,74 |
| EVL | 0,004 | 1,72 |
| CKAP5 | 0,002 | 1,71 |
| TINF2 | 0,024 | 1,69 |
| CD58 | 0,003 | 1,68 |
| PTCHD2 | 0 | 1,68 |
| **PTPRE** | **0,001** | **1,67** |
| EIF2S3 | 0,001 | 1,66 |
| TIAL1 | 0,001 | 1,65 |
| FAM13A | 0,001 | 1,64 |
| USP11 | 0,003 | 1,64 |
| **FMNL2** | **0,001** | **1,62** |
| HNRNPA2B1 | 0,014 | 1,62 |
| **BCAR1** | **0,002** | **1,61** |
| CPNE1 | 0,003 | 1,61 |
| EEF1A1 | 0,013 | 1,61 |
| FADS1 | 0,002 | 1,61 |
| MAGED2 | 0,001 | 1,61 |
| PPID | 0 | 1,61 |
| SUGP2 | 0,001 | 1,61 |

| | | |
|---|---|---|
| * FC- fold change of geometric mean intensities of classes; in bold PTPRE. FMNL2, BCAR1, proteins presented from 2 different expression clones both found significant. | | |

Of the 1452 significant 126 proteins are presented for which multiple clones were used for protein expression (with a median FC of 1.38; 3 of 126 have FC<0.8 - lower at T-early), the top 24 (FC≥1.5) derived from the list of multiple-hits listed in Table 8 are marked bold.

**Table 8: 126 Proteins are significant when expressed from multiple clones: 24 antigenic proteins with a FC>1,5 marked bold (these 126 significant proteins are all expressed from ≥2 different expression clones - and derived from BRK sample testing; Table sorted bv FC.**

| **Protein / gene symbol** | **number of multiple clone s significant** | **FC (T3 vs Tear ly )** | **Protein / gene symbol** | **number of multiple clone s significant** | **FC (T3 vs Tear ly)** | **Protein / gene symbol** | **number of multiple clone s significant** | **FC (T3 vs Tear ly)** |
|---|---|---|---|---|---|---|---|---|
| **PTCHD2** | 2 | 1.68 | TMSB10/TMSB4X | 2 | 1.44 | COPZ1 | 2 | 1.34 |
| **USP11** | 2 | 1. 64 | USP20 | 2 | 1.44 | HSD17B10 | 2 | 1.34 |
| **HNRNPA2B1** | 2 | 1.62 | ARPP19 | 2 | 1.43 | NACA | 2 | 1.34 |
| **CPNE1** | 3 | 1.61 | CCT7 | 2 | 1.43 | NYNRIN | 2 | 1.34 |
| **EEF1A1** | 4 | 1.61 | HNRNPK | 3 | 1.42 | PCID2 | 2 | 1.34 |
| **GNAI2** | 2 | 1.6 | NRXN2 | 2 | 1.42 | PTPRA | 3 | 1.34 |
| **HNRPDL** | 4 | 1.6 | TBRG4 | 2 | 1.42 | RNF216 | 2 | 1.34 |
| **DRG1** | 2 | 1.59 | APEX1 | 2 | 1.41 | TMEM132A | 2 | 1.34 |
| **PKM** | 2 | 1.57 | FAM65A | 2 | 1.41 | WBP2 | 3 | 1.34 |
| **CAPZA2** | 2 | 1.56 | MICAL1 | 2 | 1.41 | ARPC1A | 2 | 1.33 |
| **ESYT1** | 2 | 1.55 | NUDCD3 | 2 | 1.41 | EIF3C/EIF 3CL | 2 | 1.33 |
| **ACD** | 2 | 1.54 | PNMA1 | 2 | 1.41 | GTF2IRD1 | 2 | 1.33 |
| **GPSM1** | 3 | 1.54 | SPINT2 | 2 | 1.41 | HINT1 | 2 | 1.33 |
| **ELAVL3** | 2 | 1.53 | UBA1 | 2 | 1.41 | NAE1 | 2 | 1.33 |
| **FAM131A** | 2 | 1.53 | ULK1 | 2 | 1.41 | RPL19 | 2 | 1.33 |
| **AKR1B1** | 2 | 1.52 | C11orf2 | 2 | 1.4 | VIM | 3 | 1.33 |
| **LMO4** | 2 | 1.52 | CSDC2 | 2 | 1.4 | CTC1 | 2 | 1.32 |
| **RNPS1** | 2 | 1.52 | DENND5A | 2 | 1.4 | DDX39B | 2 | 1.32 |
| **ZNF132** | 2 | 1.52 | AP1B1 | 2 | 1.39 | PLCB3 | 2 | 1.32 |
| **AP2S1** | 2 | 1.51 | ATAT1 | 2 | 1.39 | CALM1 | 2 | 1.31 |
| **BTBD6** | 2 | 1.51 | BZW2 | 2 | 1.39 | DLGAP4 | 2 | 1.31 |
| **SRSF2** | 2 | 1.51 | RBM5 | 2 | 1.39 | GRK6 | 2 | 1.31 |
| **PA2G4** | 2 | 1.5 | TERF2IP | 2 | 1.39 | GTF2B | 2 | 1.31 |
| **YWHAH** | 2 | 1.5 | BCKDHA | 3 | 1.38 | RRP1 | 2 | 1.31 |
| CENPT | 2 | 1.49 | PLXNB1 | 2 | 1.38 | TPI1P2 | 2 | 1.31 |
| EIF2B4 | 2 | 1.49 | SRI | 2 | 1.38 | PLAUR | 2 | 1.3 |
| KIAA1731 | 2 | 1.49 | ADAMTS10 | 2 | 1.37 | TNC | 2 | 1.3 |
| PMM1 | 3 | 1.49 | ENO1 | 2 | 1.37 | CSNK2B | 2 | 1.29 |
| VBP1 | 3 | 1.49 | SMARCC2 | 3 | 1.37 | EPN1 | 2 | 1.29 |
| CCT3 | 2 | 1.47 | SPTAN1 | 3 | 1.37 | NELL2 | 2 | 1.29 |
| RAP1GAP | 3 | 1.47 | SRA1 | 2 | 1.37 | PPFIA1 | 3 | 1.29 |
| ACTR1B | 2 | 1.46 | ZC3H11A | 2 | 1.37 | SCAF1 | 2 | 1.29 |
| BCL11A | 2 | 1.46 | CLIC1 | 2 | 1.36 | SNX17 | 2 | 1.29 |
| EEF1G | 2 | 1.46 | NDN | 2 | 1.36 | ZNF513 | 2 | 1.29 |
| EIF4A2 | 2 | 1.46 | PFKM | 2 | 1.36 | PASK | 2 | 1.27 |
| MPST | 2 | 1.46 | POR | 2 | 1.36 | DNAJB1 | 2 | 1.25 |
| MRPL28 | 2 | 1.46 | PRDX1 | 2 | 1.36 | EIF4G2 | 2 | 1.25 |
| AURKAIP1 | 2 | 1.45 | RPL15 | 2 | 1.36 | EXOSC8 | 2 | 1.25 |
| PPP6R2 | 2 | 1.45 | PJA1 | 2 | 1.35 | FAM193B | 2 | 1.24 |
| PRKAR1B | 3 | 1.45 | RBM10 | 2 | 1.35 | RASGRP2 | 2 | 0.79 |
| PRMT1 | 2 | 1.45 | AES | 3 | 1.34 | HUWE1 | 2 | 0.77 |
| TUBA1B | 3 | 1.45 | AKR1C4 | 2 | 1.34 | SNRNP70 | 2 | 0.73 |

Comparative analysis of cases at T3 (n=10, 1 case was excluded because the T3 sample was taken 50 month before clinical diagnosis) elucidated 966 significant features (p<0,05; blocked by k-means clusters) of these 803 have been found with a ≥1.25-fold, and 594 with ≥1.5-fold higher median reactivity in cases. Subsetting the data to the 1452 features found significant in the "severe UC vs control"-serum sample study, class comparison defines an overlap of n=227 antigens, and 213 thereof are again found with significant higher reactivity in these prediagnostic UC vs control samples (Odds ratio 1.8562; 95 % CI: 1.6013 to 2.1516; chi2 p-value = 1.307e-12;).

### Example 9: Further early diagnostics

As shown in the class comparison approach and illustrated by volcano-plots, UC development seems to be associated or even driven by a general and broad autoantigenic reaction. When increased antigenic reactivities are affecting a broad spectrum of different human proteins, the elucidation of a minimal set of candidates for obtaining best classification success has been conducted using class prediction analysis of 9 paired pre-diagnostic samples. Although numbers are low the paired analysis improves statistical power and using different feature selection strategies and algorithms for classification, we came up with a correct classification of 78% - when 7 of 9 paired prediagnostic IgG profiles from patient samples close to diagnosis (4-11m; median =6m) vs about 21m prior diagnosis (sample taken 9-24m earlier then last samples available from the BRK blood bank), providing their blood as a "healthy" blood donor although auto-antibodies present. The top features in considering the 9 pairs, only 11 antigens (DCAF5, PPID, GBAS, LRP5, PKM, WNK2, POGLUT1, AAAS, LMO4, AP2S1, SLC22A17) are listed in Table 9 which have a fold change FC≥1,5 between the 2 time points studied.

**Table 9: Top features sorted by fold change derived from paired analysis of 9 pairs of patients (T3 vs T-early; FC≥1,5; univariate mis-classification rate below 0.001).**

| **ANTIGENS** | **Parametric p-value** | **t-value** | **Fold change** |
|---|---|---|---|
| DCAF5 | 0.0034474 | 3.397 | 1.81 |
| PPID | 0.0003352 | 4.476 | 1.61 |
| GBAS | 0.0008214 | 4.059 | 1.6 |
| LRP5 | 0.001681 | 3.728 | 1.58 |
| PKM | 0.000819 | 4.06 | 1.57 |
| WNK2 | 0.001018 | 3.96 | 1.55 |
| POGLUT1 | 0.0011713 | 3.895 | 1.54 |
| AAAS | 0.0001634 | 4.815 | 1.53 |
| LMO4 | 0.0025234 | 3.541 | 1.52 |
| AP2S1 | 0.0015832 | 3.756 | 1.51 |
| SLC22A17 | 0.0030934 | 3.447 | 1.5 |

Of note samples of the 2 patients who failed correct classification were among oldest and longest stored samples. Analysing the remaining 7 pairs give more convincing results by defining 56 antigens with respect to the fold change and statistics (FC≥1.6; p<5e-04, univariate misclassification rate FDR<1e-06, see Table 10; sorted by FC for 100% correct classification of the samples closest to clinical diagnosis vs those collected about 2y before. Although these findings are statistically underpowered, the defined antigens would be of high interest for further validation on independent serum or plasma samples if available.

**Table 10: Top features sorted by fold change derived from paired analysis of 7 pairs of patients (T3 vs T-early; p < 5e-04 significance level and univariate mis-classification rate below 1e-06).**

| **ANTIGENS** | **Parametric p-value** | **t-value** | **Fold change** |
|---|---|---|---|
| EVL | 0.0002057 | 4.818 | 2.1 |
| HNRNPA2B1 | 0.0000997 | 5.184 | 2.1 |
| CD58 | 0.0000735 | 5.34 | 2.06 |
| EEF1A1 | 0.0004424 | 4.439 | 2.01 |
| DRG1 | 0.0000945 | 5.211 | 1.98 |
| DHX8 | 0.0001773 | 4.893 | 1.95 |
| GNAI2 | 0.0001624 | 4.936 | 1.95 |
| ALKBH2 | 0.0001302 | 5.048 | 1.95 |
| BCAR1 | 0.0000853 | 5.264 | 1.93 |
| TIAL1 | 0.0001786 | 4.889 | 1.92 |
| FADS1 | 0.0000979 | 5.193 | 1.92 |
| EIF2S3 | 0.0001117 | 5.126 | 1.91 |
| PTCHD2 | 0.0000894 | 5.24 | 1.91 |
| LAMTOR1 | 0.000414 | 4.471 | 1.89 |
| RNF10 | 0.0003557 | 4.546 | 1.89 |
| FAM209B | 0.0003529 | 4.55 | 1.89 |
| CDCA4 | 0.0002964 | 4.636 | 1.89 |
| FAM13A | 0.000187 | 4.866 | 1.89 |
| SUGP2 | 0.0000849 | 5.266 | 1.89 |
| CPNE1 | 0.0003801 | 4.513 | 1.88 |
| VWF | 0.0003278 | 4.586 | 1.88 |
| GOT1 | 0.0000907 | 5.232 | 1.88 |
| ZNF84 | 0.0002809 | 4.663 | 1.87 |
| RUNDC3A | 0.0001665 | 4.924 | 1.87 |
| FMNL2 | 0.0001064 | 5.151 | 1.87 |
| RNASEK | 0.0001831 | 4.876 | 1.86 |
| C17orf62 | 0.0001416 | 5.006 | 1.84 |
| FAM131A | 0.0004892 | 4.389 | 1.83 |
| MINK1 | 0.000285 | 4.656 | 1.83 |
| SH3BGRL3 | 0.0001401 | 5.011 | 1.83 |
| SIRT6 | 0.0002133 | 4.8 | 1.81 |
| CKB | 0.0002853 | 4.655 | 1.8 |
| PLXNB2 | 0.0004156 | 4.47 | 1.78 |
| CCT3 | 0.0003019 | 4.627 | 1.78 |
| UBE2L3 | 0.0002807 | 4.663 | 1.78 |
| USP28 | 0.0001105 | 5.132 | 1.77 |
| TTC19 | 0.0001416 | 5.006 | 1.76 |
| EIF4A2 | 0.0004477 | 4.433 | 1.75 |
| FTSJD2 | 0.0003739 | 4.522 | 1.75 |
| R3HDM1 | 0.0004045 | 4.483 | 1.74 |
| PPID | 0.0004417 | 4.44 | 1.73 |
| WNK2 | 0.000428 | 4.455 | 1.73 |
| HSP90AB1 | 0.0001805 | 4.883 | 1.73 |
| PDHA1 | 0.0004512 | 4.429 | 1.72 |
| KIAA1731 | 0.0003517 | 4.552 | 1.72 |
| RPL36A | 0.0002 | 4.832 | 1.72 |
| DST | 0.0001255 | 5.067 | 1.71 |
| CAPZA2 | 0.0001205 | 5.087 | 1.71 |
| EIF3G | 0.0004173 | 4.468 | 1.7 |
| SRSF2 | 0.0003381 | 4.571 | 1.7 |
| DYNC1I2 | 0.0003514 | 4.552 | 1.68 |
| NARS2 | 0.0002444 | 4.732 | 1.67 |
| ZCCHC11 | 0.0004672 | 4.412 | 1.65 |
| GTF2I | 0.0004434 | 4.438 | 1.65 |
| MSL1 | 0.0004614 | 4.418 | 1.61 |
| AAAS | 0.0004851 | 4.394 | 1.6 |

In respect to "blood donations and transfusion medicine", one could speculate that blood derivatives might be causative for unwanted side reactions in recipients using blood derivatives form such donors. Exclusion of blood donors (as done today for exclusion allergic persons by a questionnaire) or exclusion of obtained donations based on autoantibody profiles needs further studies, however might not be feasible from an organizational and economic point.

### Example 10: Comparative analysis of AA in severe UC (serum) vs pre-diagnostic UC (plasma)

For evaluating the quality and reliability of antibody-profiles elucidated in both the 1) "severe-UC-case /control" and 2) the "pre-diagnostics UC samples "T-early vs T3- at DX" study, we considered from 1) both the 697 and 481 antigen-lists for which different filtering criteria were set in BRBAT when conducting the class comparison analysis. Thus for the 697-list a number of 6230 and for the 481-list 16105 features passed filtering and were considered in class comparison.

Subsetting the data-set 2 ("pre-diagnostics UC") to the 697 and conducting class comparison of "cases at T3" vs "controls" elucidated a 244 significant antigens (Odds ratio 4.2758; 95 % CI: 3.5914 to 5.0907; chi2 p-value < 2.2e-16). Of these 53 have a fold change ≥1.5; p<0,05) and listed proteins annotated by gene symbol are given in Table 11 sorted by fold change - highest on top; also the antigenic reactivity against E.coli lysates annotated as "positive controls" in tables had diagnostic predictability.

**Table 11: Top features sorted by fold change derived from comparative analysis of pre-diagnostic samples (FC>=1.5; p<0.05) subset to features significant in severe UC vs controls;**

| **Antigen** | **Parametric p-value** | **Fold-change** |
|---|---|---|
| COA5 | 0.0004034 | 1.96 |
| YES1 | 0.003192 | 1.94 |
| MCM3AP | 0.0204938 | 1.92 |
| RANBP1 | 0.0024285 | 1.87 |
| CKAP5 | 0.0418266 | 1.87 |
| FMNL1 | 0.0247211 | 1.85 |
| MATR3 | 0.0081692 | 1.84 |
| KHDRBS1 | 0.003446 | 1.82 |
| TRAPPC4 | 0.0037392 | 1.81 |
| TMEM59L | 0.0385933 | 1.77 |
| CCT2 | 0.0208834 | 1.75 |
| PSMC2 | 0.0034693 | 1.74 |
| SEC13 | 0.0010031 | 1.73 |
| NARFL | 0.0165734 | 1.71 |
| DBN1 | 0.0174876 | 1.7 |
| A1BG | 0.006369 | 1.69 |
| ARF5 | 0.0007357 | 1.66 |
| E.coli lysate | 0.0357453 | 1.66 |
| NARS2 | 0.0044935 | 1.65 |
| GBA2 | 0.007824 | 1.65 |
| ZBTB45 | 0.0034101 | 1.62 |
| BZRAP1 | 0.0142004 | 1.62 |
| EPB42 | 0.0147668 | 1.62 |
| ME3 | 0.0150876 | 1.61 |
| SERPINF1 | 0.0166999 | 1.6 |
| HID1 (C17orf28) | 0.0113792 | 1.59 |
| ANK2 | 0.0061061 | 1.58 |
| TINAGL1 | 0.0133366 | 1.57 |
| UBXN4 | 0.0189753 | 1.57 |
| XRCC6 | 0.0034957 | 1.56 |
| FLAD1 | 0.0106224 | 1.56 |
| TRIM27 | 0.0157778 | 1.56 |
| METTL3 | 0.0038496 | 1.55 |
| NDUFS7 | 0.0095324 | 1.55 |
| NDRG2 | 0.0098253 | 1.55 |
| SULT1A3/SULT1A4 | 0.0141434 | 1.55 |
| HMGA1 | 0.0117297 | 1.54 |
| CDK9 | 0.0134067 | 1.53 |
| KIAA1731 | 0.0188157 | 1.53 |
| PSMD8 | 0.0341535 | 1.53 |
| RPA3 | 0.0459989 | 1.53 |
| KIAA0195 | 0.0051826 | 1.52 |
| STT3A | 0.0078772 | 1.52 |
| DENND5A | 0.010479 | 1.52 |
| RAB14 | 0.0319998 | 1.52 |
| PTCHD2 | 0.0076875 | 1.51 |
| DST | 0.0083162 | 1.51 |
| HSP90AB1 | 0.0268583 | 1.51 |
| HSP90AB1 | 0.0307706 | 1.51 |
| VPS35 | 0.0323628 | 1.51 |
| DTX1 | 0.0363849 | 1.51 |
| EIF4G3 | 0.0081684 | 1.5 |
| SORBS2 | 0.02965 | 1.5 |

| | | |
|---|---|---|
| E.coli_lysate is preferably not used according to the invention. | | |

Similarly considering the 481 antigens in data-set 2, 43 of 481 were significant. (Odds ratio 6.2277; 95 % CI: 4.4503 to 8.7152; chi2 p-value < 2.2e-16; Class comparison results in Table 12).

**Table 12: Genes which are differentially expressed in UC risk patients**

| **Parametric p-value** | **FDR** | **Geom mean of intensities in class 1** | **Geom mean of intensities in class 2** | **Fold-change** | **Symbol** |
|---|---|---|---|---|---|
| 0,0418266 | 0,402 | 1880,4 | 1005,17 | 1,87 | CKAP5 |
| 0,0247211 | 0,402 | 1601,57 | 863,61 | 1,85 | FMNL1 |
| 0,0208834 | 0,402 | 2888,1 | 1651,22 | 1,75 | CCT2 |
| 0,007824 | 0,402 | 4966,06 | 3004,14 | 1, 65 | GBA2 |
| 0,0150876 | 0,402 | 3034,33 | 1882,77 | 1, 61 | ME 3 |
| 0,0166999 | 0,402 | 1342, 1 | 836,97 | 1, 6 | SERPINF1 |
| 0,0166264 | 0,402 | 447,12 | 280,73 | 1,59 | MAGED2 |
| 0,0061061 | 0,402 | 4947,65 | 3126,5 | 1,58 | ANK2 |
| 0,0106224 | 0,402 | 1670,24 | 1068,82 | 1,56 | FLAD1 |
| 0,0038496 | 0,402 | 2179, 9 | 1409,36 | 1,55 | METTL3 |
| 0,0141434 | 0,402 | 945,76 | 611,53 | 1,55 | SULT1A3/SULT1A4 |
| 0,0151162 | 0,402 | 1781,65 | 1167,21 | 1,53 | positive control 0,3mg Q17 2 P19 |
| 0,0188157 | 0,402 | 1571,83 | 1026,11 | 1,53 | KIAA1731 |
| 0,0459989 | 0,402 | 817,63 | 535,79 | 1,53 | RPA3 |
| 0,0418086 | 0,402 | 590,59 | 392,77 | 1,5 | ZNF622 |
| 0,0213886 | 0,402 | 1012,96 | 679,47 | 1,49 | PRDM8 |
| 0,020266 | 0,402 | 1006,39 | 682,07 | 1,48 | EPS15L1 |
| 0,0340774 | 0,402 | 1602,99 | 1081,26 | 1,48 | GNAS |
| 0,0447815 | 0,402 | 1334,54 | 910,85 | 1,47 | ADK |
| 0,0493608 | 0,403 | 4697,8 | 3204,85 | 1,47 | PRRT2 |
| 0,0063966 | 0,402 | 1345,73 | 927,15 | 1,45 | C17orf108 |
| 0,0348591 | 0,402 | 824,57 | 567,77 | 1,45 | SOX11 |
| 0,0269114 | 0,402 | 1465,65 | 1021,4 | 1,43 | RHOT2 |
| 0,0474844 | 0,402 | 1932,82 | 1353,3 | 1,43 | RPS29 |
| 0,0437789 | 0,402 | 1006,79 | 709, 46 | 1,42 | DPYSL3 |
| 0,047517 | 0,402 | 1277,45 | 901,09 | 1,42 | SNRNP25 |
| 0,0254692 | 0,402 | 865,2 | 614,17 | 1,41 | C20orf27 |
| 0,0166925 | 0,402 | 1636,77 | 1169,58 | 1,4 | AP2M1 |
| 0,0202966 | 0,402 | 1241,49 | 892,61 | 1,39 | TBC1D1 |
| 0,0391193 | 0,402 | 918,12 | 662,76 | 1,39 | HES6 |
| 0,0480952 | 0,402 | 1789,9 | 1303,85 | 1,37 | ENOX1 |
| 0,043237 | 0,402 | 1318,38 | 974,23 | 1,35 | TCTN3 |
| 0,0318967 | 0,402 | 2390,23 | 1791,51 | 1,33 | PSMB1 |
| 0,0418081 | 0,402 | 1018,17 | 790,21 | 1,29 | LCMT1 |

When considering the 697 antigens and conducting class comparison of "T-early vs T3- at DX" (paired analysis) of data-set 2 "pre-diagnostics UC", 210 antigens are significant (Odds ratio 3.4231; 95 % CI: 2.8593 to 4.0980; chi2 p-value < 2.2e-16; Of these 25 have a fold change ≥1.5 & p<0,05) and listed proteins annotated by gene symbol are given in Table 13). On top of these list sorted by FC antigenic reactivity at T3 against DCAF5 (DDB1 And CUL4 Associated Factor 5) is highest (FC=1,8); next CKAP5 known as *Cytoskeleton Associated Protein* 5 has a fold change of FC=1,7 and is also known as *Colonic And Hepatic Tumor Overexpressed Gene Protein.* In line with effecting antigen processing HSP90A1 (FC=1,5) is listed as top10^{th} (ranked by p-value).

**Table 13: Top features sorted by fold change derived from comparative analysis of pre-diagnostic samples - 9 pairs of patients (T3 vs T-early: FC ≥1.5; p<0.05) subset to features significant in severe UC vs controls.**

| **Symbol** | **Parametric p-value** | **FC (class T3 /class Tearly)** |
|---|---|---|
| DCAF5 | 0.0034474 | 1.81 |
| CKAP5 | 0.0015792 | 1.71 |
| PTCHD2 | 0.0004463 | 1.68 |
| USP11 | 0.0030261 | 1.64 |
| FMNL2 | 0.0008524 | 1.62 |
| C17orf62 | 0.000902 | 1.6 |
| MINK1 | 0.0012855 | 1.6 |
| RUNDC3A | 0.0016805 | 1.6 |
| ALKBH2 | 0.0046577 | 1.59 |
| KLC4 | 0.0161317 | 1.59 |
| RANBP1 | 0.0018488 | 1.58 |
| DPYSL3 | 0.0019474 | 1.57 |
| CKB | 0.0022278 | 1.56 |
| CAPZA2 | 0.0070737 | 1.56 |
| R3HDM1 | 0.0016836 | 1.54 |
| CAPZA2 | 0.000505 | 1.53 |
| FAM131A | 0.0064943 | 1.53 |
| HSP90AB1 | 0.0014169 | 1.52 |
| AP2S1 | 0.0015832 | 1.51 |
| DTX1 | 0.0063882 | 1.51 |
| DST | 0.0011559 | 1.5 |
| COA5 | 0.0040905 | 1.5 |
| PA2G4 | 0.0043666 | 1.5 |
| CHMP4A | 0.0058699 | 1.5 |
| RBM22 | 0.0060361 | 1.5 |

For the pre-diagnostic list of 1452 significant features 14983 features on arrays passed filtering criteria for data analysis. Intersecting this 1452 from "pre-diagnostic UC" with the 481 antigens found significant in the data-set 1 ("severe UC" vs controls) again resulted in 196 / 481 common antigens which is highly significant (Odds ratio 6.4088, 95 % CI: 5.3011 to 7.7479, chi2 p-value < 2.2e-16). From paired analysis excluding the 2 patients failed classification the number of significant antigens roughly doubles to 2612 (Table 14); intersecting these with the severe-UC vs controls derived 697 again gives a highly significant overlaps of 286 (41%) antigens (Odds ratio 3.7866; 95 % CI: 3.2355 to 4.4315, chi2 p-value < 2.2e-16).

**Table 14: Genes which are differentially expressed among UC risk persons**

| Parametric p-value | FDR | Geometric mean of intensities | Gene Symbol |
|---|---|---|---|
| 7,35E-05 | 0,094 | 2,06 | CD58 |
| 8,49E-05 | 0,094 | 1,89 | SUGP2 |
| 8,53E-05 | 0,094 | 1,93 | BCAR1 |
| 8,94E-05 | 0,094 | 1,91 | PTCHD2 |
| 9,07E-05 | 0,094 | 1,88 | GOT1 |
| 9,45E-05 | 0,094 | 1,98 | DRG1 |
| 9,79E-05 | 0,094 | 1,92 | FADS1 |
| 9,97E-05 | 0,094 | 2,1 | HNRNPA2B1 |
| 0,0001064 | 0,094 | 1,87 | FMNL2 |
| 0,0001105 | 0,094 | 1,77 | USP28 |
| 0,0001117 | 0,094 | 1,91 | EIF2S3 |
| 0,0001302 | 0,094 | 1,95 | ALKBH2 |
| 0,0001401 | 0,094 | 1,83 | SH3BGRL3 |
| 0,0001416 | 0,094 | 1,76 | TTC19 |
| 0,0001416 | 0,094 | 1,84 | C17orf62 |
| 0,0001624 | 0,094 | 1,95 | GNAI2 |
| 0,0001665 | 0,094 | 1,87 | RUNDC3A |
| 0,0001773 | 0,094 | 1,95 | DHX8 |
| 0,0001786 | 0,094 | 1,92 | TIAL1 |
| 0,000183 | 0,094 | 1,86 | RNASEK |
| 0,000187 | 0,094 | 1,89 | FAM13A |
| 0,0002057 | 0,094 | 2,1 | EVL |
| 0,0002133 | 0,094 | 1,81 | SIRT6 |
| 0,0002725 | 0,0974 | 1,8 | MAPT |
| 0,0002807 | 0,0974 | 1,78 | UBE2L3 |
| 0,0002809 | 0,0974 | 1,87 | ZNF84 |
| 0,000285 | 0,0974 | 1,83 | MINK1 |
| 0,0002852 | 0,0974 | 1,8 | CKB |
| 0,0002964 | 0,0974 | 1,89 | CDCA4 |
| 0,0003019 | 0,0974 | 1,78 | CCT3 |
| 0,0003278 | 0,0974 | 1,88 | VWF |
| 0,0003529 | 0,0974 | 1,89 | FAM209B |
| 0,0003557 | 0,0974 | 1,89 | RNF10 |
| 0,0003739 | 0,0974 | 1,75 | FTSJD2 |
| 0,0003801 | 0,0974 | 1,88 | CPNE1 |
| 0,000414 | 0,0974 | 1,89 | LAMTOR1 |
| 0,0004156 | 0,0974 | 1,78 | PLXNB2 |
| 0,0004424 | 0,0974 | 2,01 | EEF1A1 |
| 0,0004477 | 0,0974 | 1,75 | EIF4A2 |
| 0,0004892 | 0,0974 | 1,83 | FAM131A |
| 0,0005132 | 0,0974 | 1,77 | MAGED2 |
| 0,0005293 | 0,0974 | 1,78 | BTBD6 |
| 0,0005437 | 0,0974 | 1,81 | STMN3 |
| 0,0005764 | 0,0974 | 1,91 | USP11 |
| 0,0005979 | 0,0974 | 1,75 | GBAS |
| 0,0006174 | 0,0974 | 1,87 | ESYT1 |
| 0,0006207 | 0,0974 | 1,85 | PTPRE |
| 0,00063 | 0,0974 | 1,86 | CAPZA2 |
| 0,000673 | 0,0974 | 1,77 | PHC2 |
| 0,0006747 | 0,0974 | 1,76 | ALDH1B1 |
| 0,0006848 | 0,0977 | 1,79 | RBM11 |
| 0,000844 | 0,0979 | 1,81 | COMMD7 |
| 0,0008883 | 0,0979 | 1,77 | TRO |
| 0,0009487 | 0,0993 | 2,14 | SULT1A3/SULT1A4 |
| 0,0009593 | 0,0993 | 1,75 | RANBP1 |
| 0,001107 | 0,1 | 1,78 | CEP57 |
| 0,0012629 | 0,105 | 1,76 | CLN5 |
| 0,0016121 | 0,114 | 1,76 | MLST8 |
| 0,0017464 | 0,114 | 1,75 | NEUROD2 |
| 0,0020232 | 0,114 | 2,07 | DCAF5 |
| 0,0023147 | 0,114 | 1,8 | YWHAH |
| 0,002376 | 0,114 | 1,76 | RAB34 |
| 0,0023841 | 0,114 | 1,79 | RFC2 |
| 0,0025098 | 0,114 | 1,83 | RELA |
| 0,0025361 | 0,114 | 1,92 | KLC4 |
| 0,0036667 | 0,125 | 1,8 | DNTTIP2 |
| 0,0037126 | 0,126 | 1,82 | ACD |
| 0,0044997 | 0,132 | 1,96 | E.coli_0.025 1 G20 |
| 0,0047407 | 0,135 | 1,77 | CRYM |
| 0,0048499 | 0,135 | 1,78 | ZNF132 |
| 0,0051686 | 0,136 | 1,82 | LOC494127 |
| 0,0056499 | 0,14 | 1,78 | GSTM3 |
| 0,0063702 | 0,146 | 1,79 | GPSM1 |
| 0,0087644 | 0,157 | 1,99 | LAMC1 |
| 0,0088515 | 0,157 | 1,77 | ZNF362 |
| 0,0096914 | 0,159 | 1,83 | AGXT2L2 |
| 0,0122776 | 0,174 | 1,96 | TINF2 |

Selecting these features with FC ≥1.5 & p<0,05 in both serum and plasma - severe and pre-clinical settings, respectively - obtained 25 common antigens listed in Table 15.

**Table 15: Common 25 antigens sorted by fold change derived from comparative analysis of pre-diagnostic samples - 7 pairs of patients (T3 vs T-early) and features significant in severe UC vs controls - when FC ≥ 1.5 in both settings.**

| **Symbol** | **FC (class T3 /class Tearly)** | **FC (severe UC /controls)** |
|---|---|---|
| KLC4 | 1.92 | 1.51 |
| PTCHD2 | 1.91 | 1.61 |
| KIAA1731 | 1.72 | 1.57 |
| DST | 1.71 | 1.51 |
| DPYSL3 | 1.71 | 1.51 |
| PRDM8 | 1.68 | 1.58 |
| NARS2 | 1.67 | 1.57 |
| USP20 | 1.66 | 1.50 |
| CD81 | 1.65 | 1.57 |
| CENPT | 1.64 | 1.58 |
| WDR77 | 1.62 | 1.52 |
| UBXN4 | 1.62 | 1.73 |
| FMNL1 | 1.60 | 1.52 |
| AP2S1 | 1.60 | 1.72 |
| CHMP4A | 1.60 | 1.57 |
| TCTN3 | 1.58 | 1.50 |
| CKAP5 | 1.58 | 1.51 |
| PHF17 | 1.56 | 1.60 |
| GALK2 | 1.55 | 1.51 |
| LMO2 | 1.55 | 1.51 |
| SERPINF1 | 1.55 | 1.64 |
| DCX | 1.54 | 1.53 |
| CHD3 | 1.52 | 1.59 |
| CD99 | 1.51 | 1.84 |
| TMED3 | 1.50 | 1.51 |

From pre-diagnostic and severe UC study settings a considerable number of 286 antigens is common in both setting which is highly significant like results above; thus we conclude that the findings and listed antigens defined in both comparative "case /control" studies of severe-UC and pre-diagnostics UC are very reliable.

### Example 11: Correlation of marker selection method with AUC values

In order to further validate the used autoantibody marker selection process with clinical data, the AUC values for a selected group of markers (of tables 10 and 15) were determined (See also Fig. 1C). The results given in table 16 show that the marker that have been selected for pre-diagnosis fare exceptionally well with samples from pre-diagnosis cases and the markers that have been selected for UC diagnosis show good AUC results when testing samples from severe UC patients. These markers are the most preferred of the invention, in particular in addition with those of table 13, especially DCAF5.

**Table 16: AUC values**

| Name | Severe UC AUC (LMU samples) | Pre-diagnosis AUC (Bavarian blood bank samples) |
|---|---|---|
| RNASEK | 0,622 | 0,939 |
| CD58 | 0,599 | 0,939 |
| VWF | 0,672 | 0,898 |
| CDCA4 | 0,665 | 0,878 |
| RNF10 | 0,648 | 0,878 |
| EEF1A1 | 0,624 | 0,878 |
| DRG1 | 0,619 | 0,878 |
| MINK1 | 0,726 | 0,857 |
| CAPZA2 | 0,708 | 0,857 |
| CPNE1 | 0,671 | 0,857 |
| EIF2S3 | 0,664 | 0,857 |
| FADS1 | 0,660 | 0,857 |
| EVL | 0,641 | 0,857 |
| DHX8 | 0,632 | 0,857 |
| EIF4A2 | 0,606 | 0,857 |
| LAMTOR1 | 0,692 | 0,837 |
| HNRNPA2B1 | 0,602 | 0,837 |
| SIRT6 | 0,531 | 0,837 |
| KIAA1731 | 0,741 | 0,816 |
| FMNL2 | 0,698 | 0,816 |
| GNAI2 | 0,676 | 0,816 |
| FAM209B | 0,625 | 0,816 |
| TTC19 | 0,608 | 0,816 |
| SRSF2 | 0,550 | 0,816 |
| PHF17 | 0,733 | 0,796 |
| CKB | 0,728 | 0,796 |
| MSL1 | 0,706 | 0,796 |
| RUNDC3A | 0,689 | 0,796 |
| FAM13A | 0,682 | 0,796 |
| BCAR1 | 0,668 | 0,796 |
| GOT1 | 0,594 | 0,796 |
| KLC4 | 0,757 | 0,776 |
| USP20 | 0,752 | 0,776 |
| AP2S1 | 0,743 | 0,776 |
| GALK2 | 0,739 | 0,776 |
| PRDM8 | 0,727 | 0,776 |
| WDR77 | 0,727 | 0,776 |
| ALKBH2 | 0,707 | 0,776 |
| R3HDM1 | 0,695 | 0,776 |
| RPL36A | 0,688 | 0,776 |
| FAM131A | 0,684 | 0,776 |
| ZNF84 | 0,682 | 0,776 |
| DYNC1I2 | 0,676 | 0,776 |
| SUGP2 | 0,666 | 0,776 |
| CCT3 | 0,660 | 0,776 |
| EIF3G | 0,647 | 0,776 |
| TIAL1 | 0,636 | 0,776 |
| USP28 | 0,628 | 0,776 |
| SERPINF1 | 0,760 | 0,755 |
| DST | 0,725 | 0,755 |
| LMO2 | 0,712 | 0,755 |
| CENPT | 0,711 | 0,755 |
| HSP90AB1 | 0,705 | 0,755 |
| C17orf62 | 0,703 | 0,755 |
| NARS2 | 0,689 | 0,755 |
| PPID | 0,683 | 0,755 |
| PLXNB2 | 0,678 | 0,755 |
| WNK2 | 0,674 | 0,755 |
| FTSJD2 | 0,647 | 0,755 |
| PDHA1 | 0,584 | 0,755 |
| GTF2I | 0,555 | 0,755 |
| PTCHD2 | 0,713 | 0,735 |
| DPYSL3 | 0,712 | 0,735 |
| TCTN3 | 0,707 | 0,735 |
| UBE2L3 | 0,677 | 0,735 |
| ZCCHC11 | 0,529 | 0,735 |
| CHD3 | 0,751 | 0,714 |
| CD81 | 0,737 | 0,714 |
| TMED3 | 0,727 | 0,714 |
| CHMP4A | 0,709 | 0,714 |
| SH3BGRL3 | 0,680 | 0,714 |
| UBXN4 | 0,729 | 0,694 |
| AAAS | 0,673 | 0,694 |
| FMNL1 | 0,732 | 0,673 |
| CKAP5 | 0,712 | 0,673 |
| DCX | 0,749 | 0,653 |
| CD99 | 0,742 | 0,653 |

## Claims

1. A method of predicting emergence of colitis ulcerosa, detecting a predisposition to colitis ulcerosa or of diagnosing colitis ulcerosa in a subject comprising providing a sample from the subject, and detecting autoantibodies specific of colitis ulcerosa of the subject in the sample.

2. The method of claim 1, wherein the subject has not been diagnosed of suffering from colitis ulcerosa and the method is a prediction of emergence of colitis ulcerosa or a detection of a predisposition to colitis ulcerosa.

3. The method of claim 1 or 2, wherein the autoantibody detection is a determination of autoantibody amounts in comparison to antibody amounts in healthy subjects that did not develop colitis ulcerosa, preferably did not detect colitis ulcerosa within at least a year.

4. The method of any one of claims 1 to 3, wherein the autoantibody determination comprises binding to the antigens and detecting binding events, preferably wherein the antigens are immobilized on a solid surface.

5. The method of claim 4, wherein autoantibodies bound to an antigen of colitis ulcerosa are detected, preferably by a label, in particular by an optical label such as a fluorescence label.

6. The method of any one of claims 1 to 5, wherein the autoantibodies are IgG antibodies.

7. The method of any one of claims 1 to 6, wherein the antigens of colitis ulcerosa are selected by testing antibody-binding of patient derived immunoglobulins against antigens by multiplexed analysis.

8. The method of any one of claims 1 to 7, wherein the antigens of colitis ulcerosa are selected from antigens that are bound by autoantibodies of individuals that did develop colitis ulcerosa, wherein the autoantibodies bound to the antigens are collected at a time where the individuals did not yet suffer from colitis ulcerosa.

9. The method of any one of claims 1 to 8, wherein the antigens are selected from antigens of the group consisting of HNRNPA2B1, EVL, DCAF5, RNASEK, CD58, VWF, CDCA4, RNF10, EEF1A1, DRG1, MINK1, CAPZA2, CPNE1, EIF2S3, FADS1, DHX8, EIF4A2, LAMTOR1, SIRT6, KIAA1731, FMNL2, GNAI2, FAM209B, TTC19, SRSF2, PHF17, CKB, MSL1, RUNDC3A, FAM13A, BCAR1, GOT1, KLC4, USP20, AP2S1, GALK2, PRDM8, WDR77, ALKBH2, R3HDM1, RPL36A, FAM131A, ZNF84, DYNC1I2, SUGP2, CCT3, EIF3G, TIAL1, USP28, SERPINF1, DST, LMO2, CENPT, HSP90AB1, C17orf62, NARS2, PPID, PLXNB2, WNK2, FTSJD2, PDHA1, GTF2I, PTCHD2, DPYSL3, TCTN3, UBE2L3, ZCCHC11, CHD3, CD81, TMED3, CHMP4A, SH3BGRL3, UBXN4, AAAS, FMNL1, CKAP5, DCX, CD99 (Table 16).

10. The method of any one of claims 1 to 8, wherein the antigens are selected from antigens of the group consisting of combined Tables 2-16.

11. The method of claim 10 comprising detecting antibodies against a selection of at least 2 or at least 20 % of the antigens selected from any one of tables 2 to 16 in a subject, comprising the step of detecting antibodies binding said antibodies in a sample of the subject.

12. The method of claim 10 or 11, wherein the risk of developing colitis ulcerosa is determined and the antigens are selected from any one of tables 7, 8, 9, 10, 11, 12, 13, 14, 15, 16 or of combined tables 7-16; or wherein colitis ulcerosa is diagnosed and the antigens are selected from any one of tables 3, 4, 5, 6 or of combined tables 3-6, especially preferred wherein severity of colitis ulcerosa is determined by comparing with autoantibody amounts in the sample against the antigens of table 6; especially preferred wherein the severity according to the CAS score is estimated.

13. The method of any one of claims 1 to 12, wherein the step of detecting antibodies binding said antigens comprises comparing said detection signal with detection signals of a healthy control and comparing said detection signals, wherein an increase in the detection signal indicates colitis ulcerosa or a risk thereof, except for antibodies against antigens RASGRP2, HUWE1, SNRNP70, STMN4, wherefore a decrease indicates colitis ulcerosa or a risk thereof; and/or
wherein the step of detecting antibodies binding said antigens comprises comparing said detection signal with detection signals of one or more known colitis ulcerosa control sample, preferably wherein the control signals are used to obtain a antigen dependent signal pattern as indication classifier and the antigen dependent signals of the subject is compared with and/or fitted onto said pattern, thereby obtaining information of the diagnosed condition, and/or preferably further comparing said detection signals, wherein a detection signal from the sample of the subject in amount of at least 60%, preferably at least 80%, of the colitis ulcerosa control indicates colitis ulcerosa; or b) wherein a detection signal in at least 60%, preferably at least 75%, of the used antigens indicates colitis ulcerosa or the risk thereof.

14. The method of treating a patient comprising colitis ulcerosa or being at risk of developing colitis ulcerosa, comprising detecting colitis ulcerosa or the risk thereof according to any one of claims 1 to 13 and administering a colitis ulcerosa medication, preferably an anti-inflammatory medicament.

15. A kit of diagnostic agents suitable to detect autoantibodies against any antigen or antigen combination as defined in claims 9 to 12, wherein said diagnostic agents comprise antigens suitable to bind autoantibodies in a sample, preferably wherein said diagnostic agents are immobilized on a solid support, optionally further comprising a computer-readable medium or a computer program product, comprising signal data for control samples with known conditions selected from colitis ulcerosa or risk of colitis ulcerosa, and/or calibration, training or cur-off-value data for analysing said antigens provided in the kit for diagnosing colitis ulcerosa or risk of colitis ulcerosa or distinguishing conditions selected from healthy conditions, colitis ulcerosa and risk of colitis ulcerosa; further preferred wherein the kit comprises at most 3000 diagnostic agents, preferably at most 2500 diagnostic agents, at most 2000 diagnostic agents, at most 1500 diagnostic agents, at most 1200 diagnostic agents, at most 1000 diagnostic agents, at most 800 diagnostic agents, at most 500 diagnostic agents, at most 300 diagnostic agents, at most 200 diagnostic agents, at most 100 diagnostic agents; and or further preferred wherein the kit comprises a labelled secondary antibody, preferably for detecting an Fc part of autoantibodies of the subject.
